(19) Europäisches Patentamt
European Patent Office
Office européen des brevets

(11) EP 3 902 799 B1

(12) **FASCICULE DE BREVET EUROPEEN**

(45) Date de publication et mention de la délivrance du brevet:
**27.07.2022 Bulletin 2022/30**

(21) Numéro de dépôt: **20700830.1**

(22) Date de dépôt: **17.01.2020**

(51) Classification Internationale des Brevets (IPC):
***C07D 471/08*** (2006.01) ***A61K 49/08*** (2006.01)
***A61K 49/10*** (2006.01) ***C07D 487/04*** (2006.01)
***C07F 5/00*** (2006.01)

(52) Classification Coopérative des Brevets (CPC):
**C07D 471/08; A61K 49/106; C07D 487/04**

(86) Numéro de dépôt international:
**PCT/EP2020/051142**

(87) Numéro de publication internationale:
**WO 2020/148431 (23.07.2020 Gazette 2020/30)**

(54) **COMPLEXE DE GADOLINIUM ET D'UN LIGAND CHELATEUR DERIVE DE PCTA DIASTEREOISOMERIQUEMENT ENRICHI ET PROCEDE DE SYNTHESE**

KOMPLEX AUS GADOLINIUM UND EINEM CHELATBILDENDEN LIGANDEN AUS EINEM DIASTEREOISOMERENANGEREICHERTEN PCTA UND SYNTHESEVERFAHREN

COMPLEX OF GADOLINIUM AND A CHELATING LIGAND DERIVED OF A DIASTEREOISOMERICALLY ENRICHED PCTA AND SYNTHESIS METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA**
Etats de validation désignés:
**MA TN**

(30) Priorité: **17.01.2019 FR 1900433**

(43) Date de publication de la demande:
**03.11.2021 Bulletin 2021/44**

(60) Demande divisionnaire:
**21190150.9 / 3 936 505**

(73) Titulaire: **Guerbet**
**93420 Villepinte (FR)**

(72) Inventeurs:
- **LE GRENEUR, Soizic**
**91440 BURES SUR YVETTE (FR)**
- **CHÉNEDÉ, Alain**
**17140 LAGORD (FR)**
- **CERF, Martine**
**17870 BREUIL-MAGNÉ (FR)**
- **DECRON, Stéphane**
**17230 MARANS (FR)**
- **FRANÇOIS, Bruno**
**17170 SAINT JEAN DE LIVERSAY (FR)**

(74) Mandataire: **Regimbeau**
**20, rue de Chazelles**
**75847 Paris Cedex 17 (FR)**

(56) Documents cités:
**EP-A1- 1 931 673** **WO-A1-2014/174120**
**WO-A1-2020/030618**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 902 799 B1

## Description

La présente invention a trait à un nouveau procédé de synthèse d'un complexe de gadolinium et d'un ligand chélateur dérivé de PCTA, qui permet d'obtenir de manière préférentielle les stéréoisomères dudit complexe qui présentent des propriétés physico-chimiques tout particulièrement intéressantes pour des applications en tant qu'agent de contraste dans le domaine de l'imagerie médicale, notamment pour l'Imagerie par Résonance Magnétique.

On connaît de nombreux agents de contraste à base de chélates de lanthanides (métal paramagnétique), en particulier de gadolinium (Gd), décrits par exemple dans le brevet US 4,647,447. Ces produits sont souvent rassemblés sous le terme GBCA (Gadolinium-based Contrast Agent, produits de contraste à base de gadolinium). Plusieurs produits sont commercialisés, parmi lesquels figurent des chélates macrocycliques, tels que le gadotérate de méglumine à base de DOTA (acide 1,4,7,10-tétraazacyclododécane-N,N',N",N'''-tétraacétique), le gadobutrol à base de DO3A-butrol, le gadotéridol à base de HPDO3A, ainsi que des chélates linéaires, notamment à base de DTPA (acide diéthylènetriaminepentaacétique) ou de DTPA-BMA (ligand du gadodiamide).

D'autres produits, dont certains sont en cours de développement, représentent une nouvelle génération de GBCA. Il s'agit essentiellement de complexes de chélates macrocycliques, tels que les complexes de l'acide bicyclopolyazamacrocyclocarboxylique (EP 0 438 206) ou de dérivés de PCTA (c'est-à-dire comprenant a *minima* la structure chimique de l'acide 3,6,9,15-tétraazabicyclo[9,3,1]pentadéca-1(15),11,13-triène-3,6,9-triacétique), comme décrits dans le document EP 1 931 673.

Les complexes de ligands chélateurs dérivés de PCTA décrits dans le document EP 1 931 673 ont notamment comme avantage d'être relativement faciles à synthétiser chimiquement, et, de surcroît, de présenter une relaxivité supérieure aux autres GBCA (relaxivité $r_1$ pouvant aller jusqu'à 11-12 $mM^{-1}.s^{-1}$ dans l'eau) actuellement sur le marché, cette relaxivité correspondant à l'efficacité de ces produits et donc à leur pouvoir contrastant.

Dans l'organisme, les chélates (ou complexes) de lanthanide - et notamment de gadolinium - sont en situation d'équilibre chimique (caractérisé par sa constante thermodynamique $K_{therm}$), ce qui peut conduire à une libération non souhaitée dudit lanthanide (voir équation 1 ci-après) :

$$Ch + Ln \rightleftarrows Ch\text{-}Ln$$

(équation 1)

*Equilibre chimique de complexation entre le chélate ou ligand (Ch) et le lanthanide (Ln) pour conduire au complexe Ch-Ln.*

Depuis 2006, une pathologie appelée NSF (Nephrogenic Systemic Fibrosis, fibrose néphrogénique systémique ou dermopathie fibrogénique), a été reliée, au moins en partie, à la libération de gadolinium libre dans l'organisme. Cette maladie a conduit à une alerte des autorités de santé vis-à-vis d'agents de contraste gadolinés commercialisés pour certaines catégories de patients.

Des stratégies ont donc été mises en place pour résoudre de manière complètement sécurisée le problème complexe de la tolérance chez le patient, et limiter, voire éliminer, le risque de libération de lanthanide non souhaitée après administration. Ce problème est d'autant plus délicat à résoudre que l'administration d'agents de contraste est souvent répétée, que ce soit lors d'examens de diagnostic, ou pour l'ajustement des doses et le suivi de l'efficacité d'un traitement thérapeutique.

En outre, depuis 2014 est évoqué un possible dépôt cérébral de gadolinium après administrations répétées de produits gadolinés, plus particulièrement de chélates de gadolinium linéaires, un tel dépôt n'ayant pas, ou peu, été rapporté avec les chélates macrocycliques de gadolinium, comme Dotarem® En conséquence, différents pays ont décidé soit de retirer la plupart des chélates linaires du marché, soit de limiter drastiquement leurs indications d'utilisation, compte tenu de leur stabilité estimée insuffisante.

Une stratégie de limitation du risque de libération de lanthanide dans l'organisme consiste ainsi à opter pour des complexes qui se distinguent par des stabilités thermodynamique et/ou cinétique les plus élevées possible. En effet, plus le complexe est stable, plus la quantité de lanthanide libérée au cours du temps sera limitée.

Or, les complexes de ligands chélateurs dérivés de PCTA comprenant une structure de type pyclène décrits dans le document EP 1 931 673, tout en ayant une bonne stabilité cinétique, ont, en général, une constante thermodynamique plus faible que celle des complexes des autres macrocycles dérivés du cyclène.

C'est notamment le cas du complexe de formule (II) représentée ci-après :

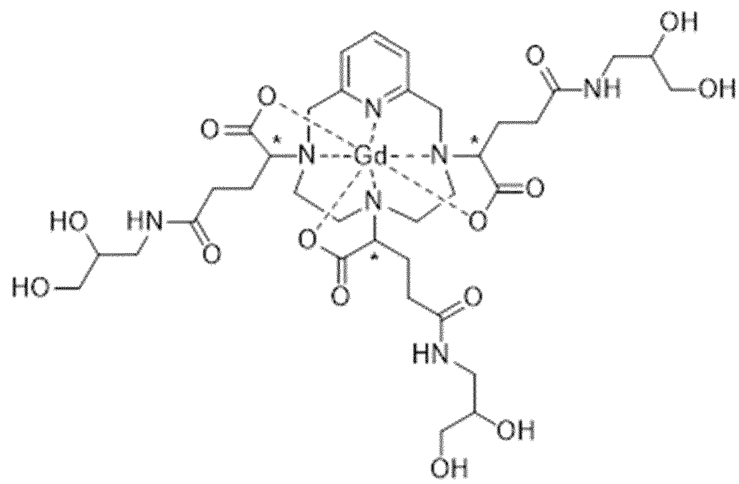

(II)

En effet, comme cela est notamment décrit dans le document WO 2014/174120, la constante d'équilibre thermodynamique correspondant à la réaction de formation du complexe de formule (II), également appelée constante de stabilité, est de $10^{14,9}$ (i.e. log ($K_{therm}$) = 14,9). A titre de comparaison, la constante de stabilité du complexe de gadolinium de l'acide 1, 4, 7, 10 tétra-azacyclododécane N, N', N", N'" tétra-acétique (DOTA-Gd), est de $10^{25,6}$ (i.e. log ($K_{therm}$) = 25,6).

Il convient toutefois de noter que le complexe de formule (II) correspond à plusieurs stéréoisomères, notamment du fait de la présence des trois atomes de carbones asymétriques situés en position $\alpha$ sur les chaînes latérales du complexe, par rapport aux atomes d'azote du macrocycle sur lesquels sont greffées lesdites chaînes latérales. Ces trois carbones asymétriques sont marqués d'un astérisque (*) dans la formule (II) représentée ci-dessus.

Ainsi, la synthèse du complexe de formule (II) telle que décrite dans le document EP 1 931 673 aboutit à l'obtention d'un mélange de stéréoisomères.

Les groupements aminopropanediol des chaînes latérales du complexe de formule (II) comportent également un carbone asymétrique. Ainsi, le complexe de formule (II) comprend au total 6 carbones asymétriques, et existe par conséquent sous la forme de 64 stéréoisomères de configuration. Toutefois, dans la suite de la description, la seule source de stéréoisomérie considérée pour une chaîne latérale donnée sera, par souci de simplification, celle correspondant au carbone asymétrique portant le groupement carboxylate, marqué d'un astérisque (*) dans la formule (II) représentée ci-dessus.

Dans la mesure où chacun de ces 3 carbones asymétriques peut être de configuration absolue R ou S, le complexe de formule (II) existe sous la forme de 8 familles de stéréoisomères, dénommées ci-après II-RRR, II-SSS, II-RRS, II-SSR, II-RSS, II-SRR, II-RSR et II-SRS. Plus précisément, selon la nomenclature usuelle en stéréochimie, le complexe de formule (II) existe sous la forme de 8 familles de diastéréoisomères.

L'emploi du terme « famille » se justifie en cela que chacune de ces familles regroupe plusieurs stéréoisomères, notamment du fait de la présence d'un carbone asymétrique au sein du groupement aminopropanediol, comme évoqué précédemment.

Néanmoins, dans la mesure où, dans la suite de la description, la stéréoisomérie liée au carbone asymétrique d'un groupement aminopropanediol donné ne sera pas considérée, on parlera indifféremment d'isomères, stéréoisomères ou encore diastéréoisomères II-RRR, II-SSS, II-RRS, II-SSR, II-RSS, II-SRR, II-RSR et II-SRS, sans préciser que chacun correspond à une famille de stéréoisomères.

Les inventeurs sont parvenus à séparer et à identifier par chromatographie en phase liquide à haute performance (CLHP, plus communément désignée par l'acronyme anglais HPLC) et par chromatographie en phase liquide à ultra haute performance (CLUHP, plus communément désignée par l'acronyme anglais UHPLC) 4 massifs ou groupes d'isomères du complexe de formule (II) obtenu selon le procédé de l'art antérieur, correspondant à 4 pics d'élution différents caractérisés par leur temps de rétention sur le chromatogramme, que l'on nommera iso1, iso2, iso3 et iso4 dans la suite de la description. En mettant en œuvre le procédé décrit dans le document EP 1 931 673, les teneurs respectives des groupes iso1, iso2, iso3 et iso4 dans le mélange obtenu sont les suivantes : 20 %, 20 %, 40 % et 20 %.

Ils ont alors découvert que ces différents groupes d'isomères présentaient des propriétés physico-chimiques distinctes, et ont déterminé que le groupe d'isomères dénommé iso4, qui comprend un mélange des isomères II-RRR et II-SSS de formules (II-RRR) et (II-SSS) représentées ci-après, s'avère être le plus intéressant en tant qu'agent de contraste pour l'imagerie médicale.

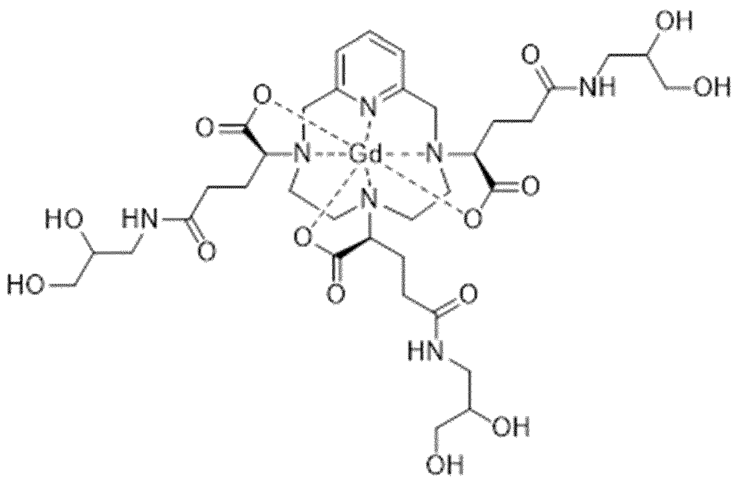

(II-SSS)

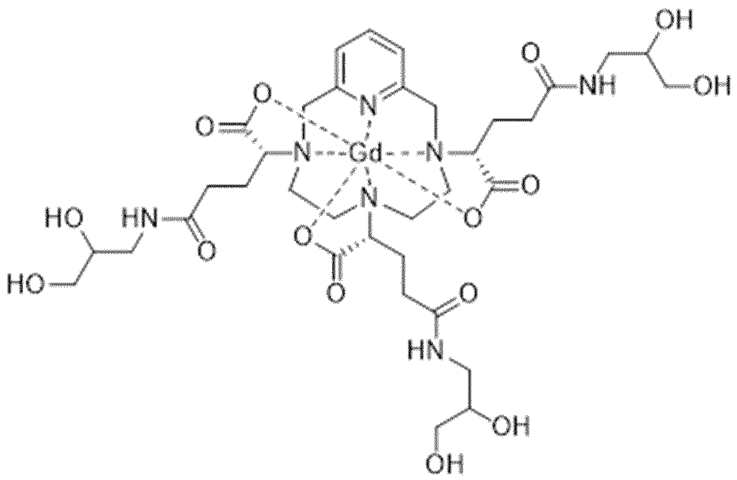

(II-RRR)

Ainsi, l'iso4 se distingue, de façon étonnante, par une stabilité thermodynamique nettement supérieure à celle du mélange de diastéréoisomères sous la forme duquel est obtenu le complexe de formule (II) en mettant en œuvre le procédé décrit dans le document EP 1 931 673. En effet, sa constante thermodynamique d'équilibre $K_{therm\ iso4}$ est égale à $10^{18,7}$ (i.e. log ($K_{therm\ iso4}$) = 18,7), cette valeur ayant été déterminée en mettant en œuvre la méthode décrite dans Pierrard et al., Contrast Media Mol. Imaging, 2008, 3, 243-252 et Moreau et al., Dalton Trans., 2007, 1611-1620.

De surcroît, l'iso4 est le groupe d'isomères qui présente la meilleure inertie cinétique (également appelée stabilité cinétique) parmi les quatre groupes isolés par les inventeurs. En effet, les inventeurs ont évalué l'inertie cinétique des quatre groupes d'isomères en étudiant leur cinétique de décomplexation en solution aqueuse acide (pH = 1,2), à 37°C. Les valeurs des temps de demi-vie ($T_{1/2}$) qui ont été déterminées pour chacun des groupes d'isomères sont indiquées dans le tableau 1 ci-dessous, le temps de demi-vie correspondant à la durée au bout de laquelle 50 % de la quantité de complexe initialement présente a été dissociée, selon la réaction de décomplexation suivante (équation 2) :

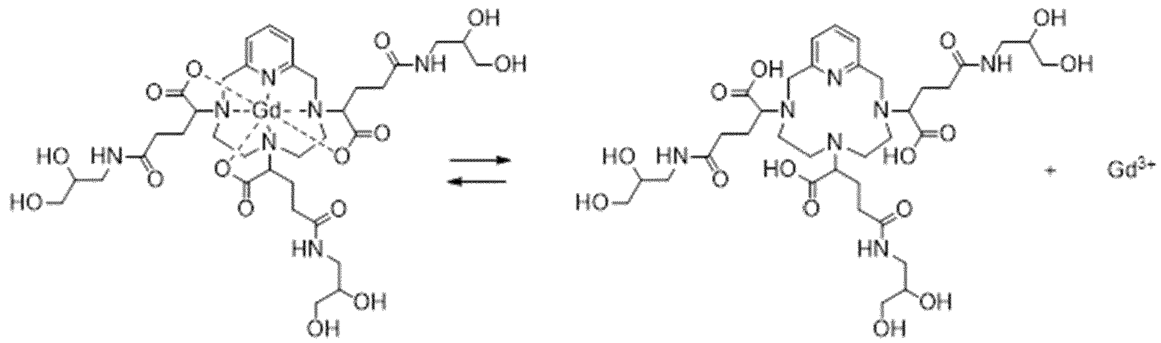

(équation 2)

Tableau 1 : cinétique de décomplexation des groupes d'isomères iso1 à iso4

| Groupes d'isomères | $T_{1/2}$ (pH 1,2 - 37 °C) |
|---|---|
| Iso1 | 18 heures |
| Iso2 | 6 heures |
| Iso3 | 8 jours |
| Iso4 | 27 jours |

A titre de comparaison, le gadobutrol ou le gadoterate, complexes macrocycliques de gadolinium, présentent respectivement une inertie cinétique de 18h et de 4 jours dans les mêmes conditions, tandis que les complexes linéaires de gadolinium comme le gadodiamide ou le gadopentetate se dissocient instantanément.

En outre, l'iso4 est plus stable d'un point de vue chimique que l'iso3 notamment. Les fonctions amides du complexe de formule (II) sont en effet susceptibles d'être hydrolysées. La réaction d'hydrolyse d'une fonction amide (équation 3) aboutit à la formation d'une impureté dicouplée, qui s'accompagne de la libération de 3-amino-1,2-propanediol. Les inventeurs ont étudié la cinétique de la réaction d'hydrolyse du complexe de formule (II) en solution aqueuse à pH 13, et observé que les fonctions amides de l'iso4 sont plus stables vis-à-vis de l'hydrolyse que celles de l'iso3.

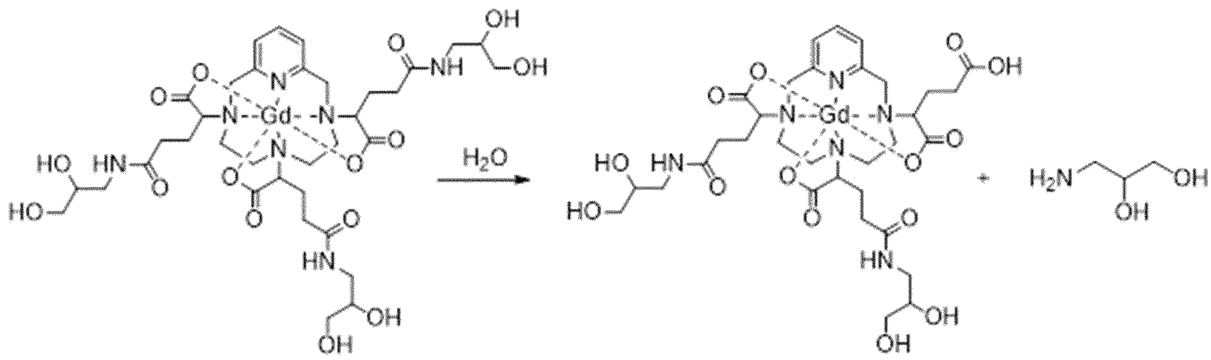


(équation 3)

En ce qui concerne la relaxivité des différents groupes d'isomères, c'est-à-dire leur efficacité en tant qu'agent de contraste, les mesures réalisées mettent en évidence un pouvoir contrastant relativement équivalent pour les groupes iso1, iso2 et iso4, et une efficacité moindre pour l'iso3 (voir tableau 2).

Tableau 2 : relaxivité des groupes d'isomères iso1 à iso4 à 37°C

| **Groupes d'isomères** | **r1 20 MHz** ($mM^{-1}.s^{-1}$) | **r1 60 MHz** ($mM^{-1}.s^{-1}$) |
|---|---|---|
| Iso1 | 12.6 | 12.5 |

(suite)

| **Groupes d'isomères** | **r1 20 MHz** ($mM^{-1}.s^{-1}$) | **r1 60 MHz** ($mM^{-1}.s^{-1}$) |
|---|---|---|
| Iso2 | 13.3 | 12.9 |
| Iso3 | 8.0 | 8.1 |
| Iso4 | 12.9 | 13.0 |

Les inventeurs sont parvenus à développer un nouveau procédé de préparation du complexe de formule (II) permettant d'obtenir préférentiellement les diastéréoisomères II-RRR et II-SSS dudit complexe, qui présentent des propriétés physico-chimiques particulièrement avantageuses. Le procédé selon l'invention comprend une étape d'enrichissement isomérique, par conversion des stéréoisomères les moins stables vers les stéréoisomères les plus stables, qui, de manière surprenante, tout en étant réalisée sur le complexe intermédiaire hexaacide et non pas sur le complexe final, permet d'obtenir très majoritairement les isomères du complexe de formule (II) les plus stables. Le procédé selon l'invention a été défini dans les revendications. Bien que la description qui suit peut à titre d'information inclure de la matière allant au-delà de la portée de l'invention, cette portée et donc aussi l'étendue de protection restent limitées à l'objet défini par les revendications.

La mise en œuvre d'un procédé qui permet d'obtenir majoritairement les diastéréoisomères d'intérêt est incontestablement avantageuse par rapport à l'alternative consistant à préparer le mélange de stéréoisomères, pour ensuite tenter de séparer les diastéréoisomères selon les techniques usuelles et ainsi isoler les stéréoisomères d'intérêt. En effet, outre le fait qu'il est plus aisé de mettre en œuvre un procédé dépourvu d'une étape de séparation de diastéréoisomères à l'échelle industrielle, l'absence de séparation permet d'une part un gain de temps considérable, et, d'autre part, d'améliorer le rendement global du procédé, en limitant autant que possible l'obtention des diastéréosiomères non désirés qui seraient *in fine* éliminés. Par ailleurs, les techniques de séparation usuelles impliquent de manière générale une utilisation abondante de solvants, qui, au-delà du coût financier, n'est pas souhaitable pour des raisons environnementales. De surcroît, la chromatographie sur silice en particulier est à éviter, compte tenu des risques pour la santé inhérents à une exposition professionnelle à la silice, classée comme cancérogène pour l'homme (groupe 1) par le Centre International de Recherche contre le Cancer.

Comme indiqué précédemment, le procédé de préparation du complexe de formule (II) mis au point par les inventeurs repose sur une étape d'enrichissement isomérique du complexe de gadolinium d'hexaacide intermédiaire de formule (I) représentée ci-après :

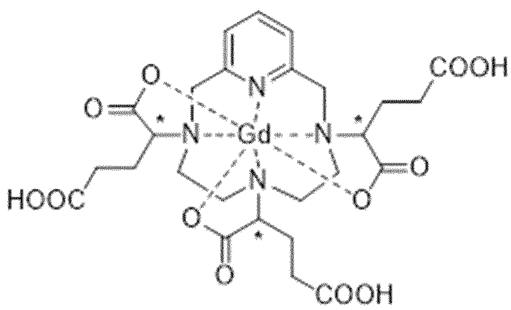

(I)

Le complexe de formule (I) correspond à plusieurs stéréoisomères, du fait de la présence des trois atomes de carbones asymétriques situés en position $\alpha$ sur les chaînes latérales du complexe, par rapport aux atomes d'azote du macrocycle sur lesquels sont greffées lesdites chaînes latérales. Ces trois carbones asymétriques sont marqués d'un astérisque (*) dans la formule (I) représentée ci-dessus.

Dans la mesure où chacun des 3 carbones asymétriques portant une fonction carboxylate peut être de configuration absolue R ou S, le complexe de formule (I) existe sous la forme de 8 stéréoisomères, dénommés ci-après I-RRR, I-SSS, I-RRS, I-SSR, I-RSS, I-SRR, I-RSR et I-SRS. Plus précisément, selon la nomenclature usuelle en stéréochimie, le complexe de formule (I) existe sous la forme de 4 paires d'énantiomères, diastéréoisomères entre elles.

Les inventeurs sont parvenus à séparer et à identifier par chromatographie en phase liquide à haute performance (CLHP, plus communément désignée par l'acronyme anglais HPLC) et par chromatographie en phase liquide à ultra haute performance (CLUHP, plus communément désignée par l'acronyme anglais UHPLC) 4 massifs ou groupes d'isomères du complexe de formule (I) obtenu selon le procédé décrit dans le document EP 1 931 673, correspondant à 4

pics d'élution différents caractérisés par leur temps de rétention sur le chromatogramme, que l'on nommera isoA, isoB, isoC et isoD dans la suite de la description.

L'isoD cristallise dans l'eau. Des analyses par diffraction des rayons X ont permis aux inventeurs de déterminer la structure cristalline de ce groupe d'isomères, et ainsi de découvrir qu'il comprend les diastéréoisomères I-RRR et I-SSS du complexe de formule (I), de formules (I-RRR) et (I-SSS) représentées ci-après.

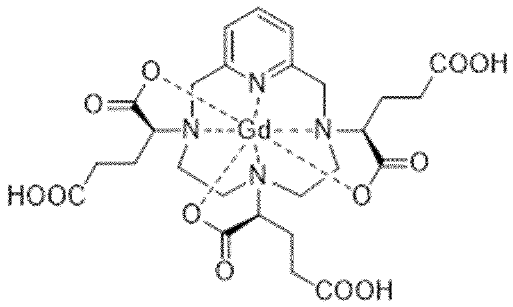

(I-SSS)

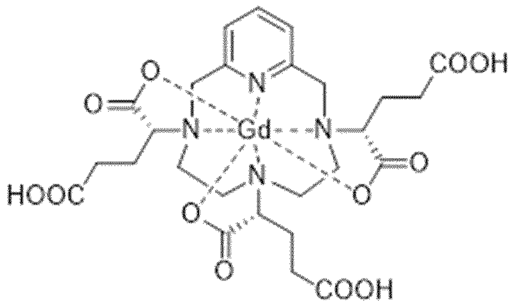

(I-RRR)

Il est à noter que les diastéréosiomères I-RRR et I-SSS du complexe de formule (I) sont énantiomères l'un de l'autre.

L'étape d'enrichissement isomérique du procédé de l'invention vise à enrichir le complexe de gadolinium d'hexaacide intermédiaire de formule (I) en isoD.

La synthèse du complexe de formule (II) implique notamment une conversion des fonctions acides carboxyliques du complexe hexaacide intermédiaire de formule (I) en fonction amide. Cette réaction d'amidification ne modifie pas la configuration absolue des trois atomes de carbones asymétriques du complexe de formule (I).

Ainsi, lorsque la réaction d'amidification est réalisée sur le complexe hexaacide de formule (I) enrichi en isoD précédemment obtenu, elle permet d'obtenir le complexe de formule (II) enrichi en iso4.

***Complexe de gadolinium d'hexaacide de formule (I)***

Le procédé selon l'invention implique donc le complexe de gadolinium d'hexaacide de formule (I) :

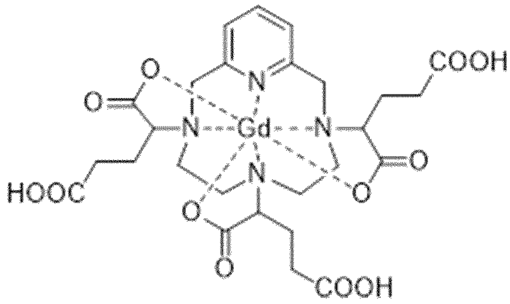

(I)

constitué d'au moins 80 % d'un excès diastéréoisomérique comprenant un mélange d'isomères I-RRR et I-SSS de formules :

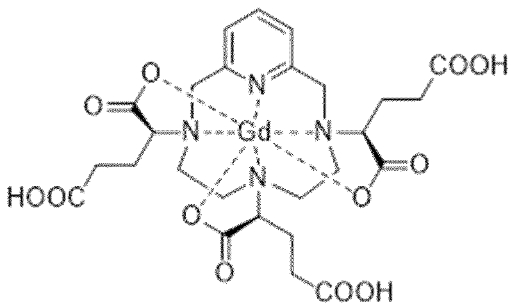

(I-SSS)

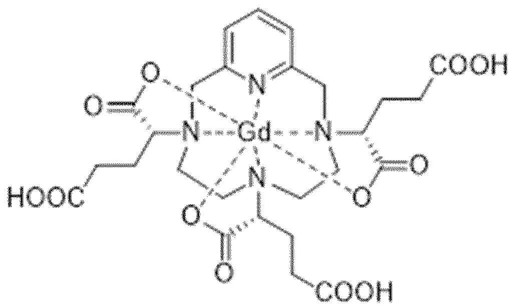

(I-RRR)

Par « excès diastéréoisomérique », on entend désigner, dans le cadre de la présente invention, et en ce qui concerne le complexe de gadolinium d'hexaacide de formule (I), le fait que ledit complexe est majoritairement présent sous la forme d'un isomère ou groupe d'isomères choisi(s) parmi les diastéréoisomères I-RRR, I-SSS, I-RRS, I-SSR, I-RSS, I-SRR, I-RSR et I-SRS. Ledit excès diastéréoisomérique est exprimé en pourcentage, et correspond à la quantité que représente l'isomère ou le groupe d'isomères majoritaire par rapport à la quantité totale du complexe de gadolinium d'hexaacide de formule (I). Il est entendu que ce pourcentage peut être aussi bien molaire que massique, dans la mesure où des isomères ont, par définition, la même masse molaire.

Dans un mode de réalisation particulier du procédé selon l'invention, le complexe de formule (I) présente au moins 85 %, notamment au moins 90 %, en particulier au moins 95 %, de préférence au moins 97 %, avantageusement au moins 98 %, plus avantageusement au moins 99 % de l'excès diastéréoisomérique comprenant le mélange d'isomères I-RRR et I-SSS.

De préférence, ledit excès diastéréoisomérique est constitué d'au moins 70 %, notamment d'au moins 80 %, avantageusement d'au moins 90 %, de préférence d'au moins 95 % du mélange d'isomères I-RRR et I-SSS.

Avantageusement, ledit excès diastéréoisomérique consiste en le mélange d'isomères I-RRR et I-SSS.

Le terme « mélange d'isomères I-RRR et I-SSS » recouvre également, par extension, le cas où seul l'un des

isomères, qu'il s'agisse du I-RRR ou du I-SSS, est présent. Toutefois, le terme « mélange d'isomères I-RRR et I-SSS » désigne préférentiellement l'ensemble des cas où chacun des isomères I-RRR et I-SSS est présent en une quantité variable mais non nulle.

Dans un mode de réalisation préféré du procédé selon l'invention, les isomères I-RRR et I-SSS sont présents au sein dudit mélange dans un rapport compris entre 65/35 et 35/65, notamment entre 60/40 et 40/60, en particulier entre 55/45 et 45/55. Avantageusement, le mélange de d'isomères I-RRR/I-SSS est un mélange racémique (50/50).

Plus particulièrement, l'excès diastéréoisomérique tel que défini précédemment correspond au pic 4 de la trace HPLC (c'est-à-dire le quatrième pic dans l'ordre d'élution et correspondant à isoD), caractérisé par un temps de rétention compris entre 33,9 et 37,5 minutes, typiquement d'environ 35,7 minutes, ladite trace étant obtenue en mettant en œuvre la méthode HPLC décrite ci-après.

Par «trace HPLC», on entend, au sens de la présente invention, le profil des concentrations mesurées par le détecteur après passage et séparation d'un mélange de composés (en l'espèce d'isomères d'un composé) sur une phase stationnaire en fonction du temps pour une composition et un débit d'éluant donné. La trace HPLC est constituée de différents pics ou massifs caractéristiques du composé ou du mélange de composés analysés.

**Méthode HPLC :**

- colonne Symmetry® C18 - 250 x 4,6 mm - 5 μm de Waters.

Il s'agit d'une colonne HPLC en phase inverse à particules sphériques de silice avec un greffage C18 (octadécyl) et dont les silanols ont été traités par des agents de coiffage (end-capped). Elle est en outre caractérisée par une longueur de 250 mm, un diamètre intérieur de 4,6 mm, une granulométrie de 5 μm, une porosité de 100 Å et un taux de carbone de 19 %.

De manière préférentielle, la phase stationnaire utilisée doit être compatible avec les phases mobiles aqueuses.

- conditions d'analyse :

| | |
|---|---|
| Echantillon | Solution aqueuse du complexe de formule (I) à 10 mg/mL |
| Température colonne | 25°C |
| Température échantillon | Température ambiante (20-25°C) |
| Débit | 1,0 mL/min |
| Volume d'injection | 20 μL |
| Détection UV | 200 nm |

- gradient phase mobile (volumique) :

| Temps (min) | % acétonitrile (100 %) | % $H_2SO_4$ (solution aqueuse à 0,1 % v/v) |
|---|---|---|
| 0 | 1 | 99 |
| 10 | 5 | 95 |
| 40 | 10 | 90 |

***Complexe de formule (II)***

Le procédé selon l'invention permet d'obtenir le complexe de formule (II) :

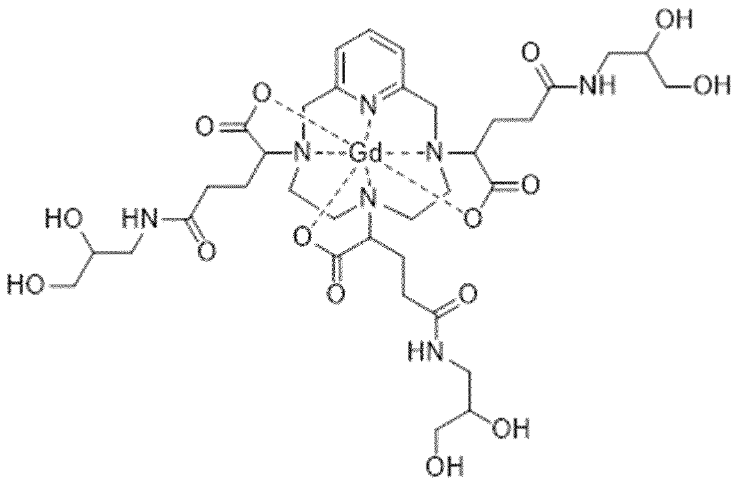

(II)

constitué d'au moins 80 % d'un excès diastéréoisomérique comprenant un mélange d'isomères II-RRR et II-SSS de formules :

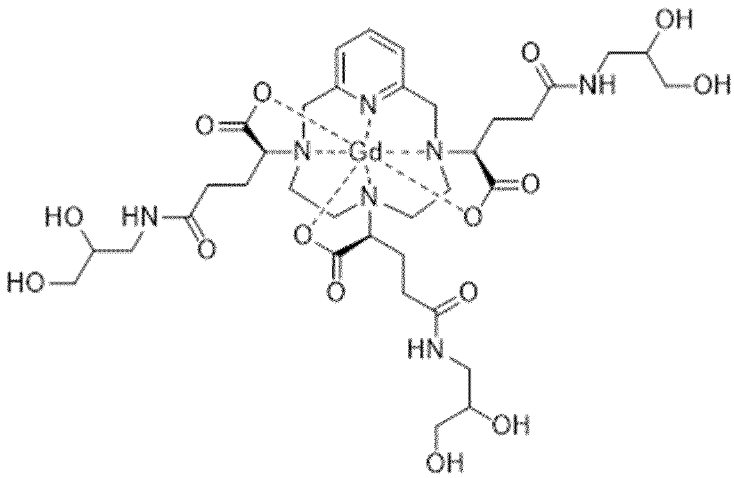

(II-SSS)

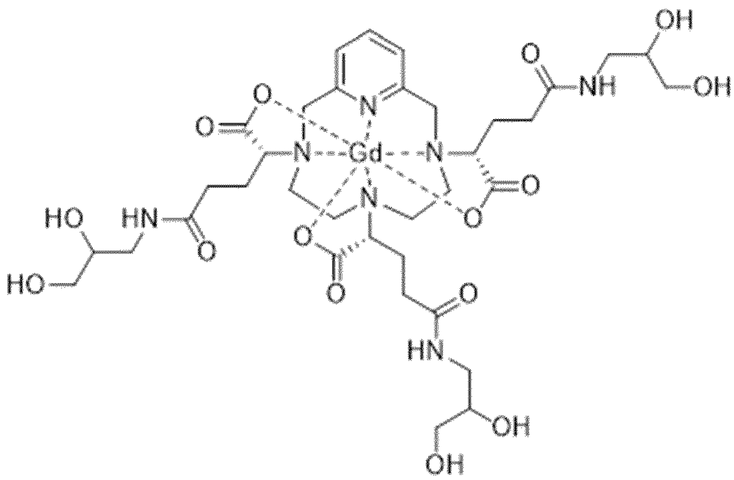

(II-RRR)

Par « excès diastéréoisomérique », on entend désigner, dans le cadre de la présente invention, et en ce qui concerne le complexe de formule (II), le fait que ledit complexe est majoritairement présent sous la forme d'un isomère ou groupe d'isomères choisi(s) parmi les diastéréoisomères II-RRR, II-SSS, II-RRS, II-SSR, II-RSS, II-SRR, II-RSR et II-SRS. Ledit excès diastéréoisomérique est exprimé en pourcentage et correspond à la quantité que représente l'isomère ou le groupe d'isomères majoritaire par rapport à la quantité totale du complexe de formule (II). Il est entendu que ce pourcentage peut être aussi bien molaire que massique, dans la mesure où des isomères ont, par définition, la même masse molaire.

Dans un mode de réalisation particulier, le complexe de formule (II) obtenu par le procédé selon l'invention présente au moins 85 %, notamment au moins 90 %, en particulier au moins 92 %, de préférence au moins 94 %, avantageusement au moins 97 %, plus avantageusement au moins 99 % de l'excès diastéréoisomérique comprenant le mélange d'isomères II-RRR et II-SSS.

De préférence, ledit excès diastéréoisomérique est constitué d'au moins 70 %, notamment d'au moins 80 %, avantageusement d'au moins 90 %, de préférence d'au moins 95 % du mélange d'isomères II-RRR et II-SSS.

Avantageusement, ledit excès diastéréoisomérique consiste en le mélange d'isomères II-RRR et II-SSS.

Le terme « mélange d'isomères II-RRR et II-SSS » recouvre également, par extension, le cas où seul l'un des isomères, qu'il s'agisse du II-RRR ou du II-SSS, est présent. Toutefois, le terme « mélange d'isomères II-RRR et II-SSS » désigne préférentiellement l'ensemble des cas où chacun des isomères II-RRR et II-SSS est présent en une quantité variable mais non nulle.

Dans un mode de réalisation préféré du procédé selon l'invention, les isomères II-RRR et II-SSS sont présents au sein dudit mélange dans un rapport compris entre 65/35 et 35/65, notamment entre 60/40 et 40/60, en particulier entre 55/45 et 45/55. Avantageusement, les isomères II-RRR et II-SSS sont présents au sein du mélange dans un rapport 50/50.

Plus particulièrement, l'excès diastéréoisomérique tel que défini précédemment correspond au pic 4 de la trace UHPLC (c'est-à-dire le quatrième massif d'isomères dans l'ordre d'élution et correspondant à iso4), caractérisé par un temps de rétention compris entre 6,0 et 6,6 minutes, typiquement d'environ 6,3 minutes, ladite trace étant obtenue en mettant en œuvre la méthode UHPLC décrite ci-après.

Par « trace UHPLC », on entend, au sens de la présente invention, le profil des concentrations mesurées par le détecteur après passage et séparation d'un mélange de composés (en l'espèce d'isomères d'un composé) sur une phase stationnaire en fonction du temps pour une composition et un débit d'éluant donné. La trace UHPLC est constituée de différents pics ou massifs caractéristiques du composé ou du mélange de composés analysés.

**Méthode UHPLC** :

- colonne CORTECS® UPLC T3 150 x 2,1 mm - 1,6 µm de Waters.

Il s'agit d'une colonne UPLC en phase inverse à particules sphériques constituées d'un noyau, préférentiellement très dur, en silice entouré d'une silice poreuse avec un greffage C18 (octadécyl) trifonctionnel, et dont les silanols ont été traités par des agents de coiffage (end-capped). Elle est en outre caractérisée par une longueur de 150 mm, un diamètre intérieur de 2,1 mm, une granulométrie de 1,6 µm, une porosité de 120 Å et un taux de carbone de 4,7 %.

De manière préférentielle, la phase stationnaire utilisée doit être compatible avec les phases mobiles aqueuses.
- conditions d'analyse :

| | |
|---|---|
| Echantillon | Solution aqueuse du complexe de formule (II) à 2,0 mg/mL |
| Température colonne | 40°C |
| Température échantillon | Température ambiante (20-25°C) |
| Débit | 0,3 mL/min |
| Volume d'injection | 1 µL |
| Détection UV | 200 nm |

- gradient phase mobile (% v /v) :

| Temps (min) | Acétonitrile (100 %) | $H_2SO_4$ (solution aqueuse à 0,0005 % v/v) |
|---|---|---|
| 0 | 1 | 99 |
| 3 | 5 | 95 |
| 12 | 10 | 90 |

Dans le procédé selon l'invention, le complexe de formule (II) est obtenu par amidification à partir du complexe de formule (I) tel que défini ci-dessus et du 3-amino-1,2-propanediol, sous forme racémique ou énantiomériquement pure, de préférence sous forme racémique.

Par « amidification », on entend, au sens de la présente invention, la réaction de conversion d'une fonction acide carboxylique en fonction amide par réaction avec une fonction amine.

Une telle réaction peut notamment être réalisée après activation des fonctions acides carboxyliques, comme cela est détaillé dans la suite de la description.

***<u>Procédé de préparation du complexe de formule (II)</u>***

La présente invention concerne donc un procédé de préparation du complexe de formule (II) comprenant les étapes successives suivantes :

**a)** Complexation de l'hexaacide de formule (III) suivante

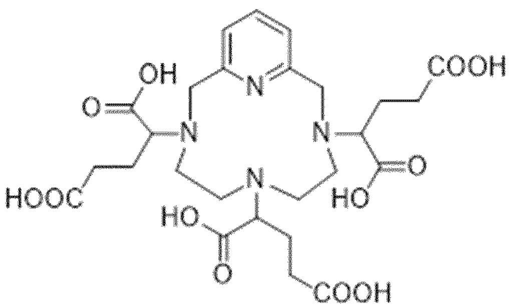

(III)

avec du gadolinium pour obtenir le complexe de gadolinium d'hexaacide de formule (I) telle que définie précédemment,

**b)** Isomérisation par chauffage du complexe de gadolinium d'hexaacide de formule (I) dans une solution aqueuse

à pH compris entre 2 et 4, pour obtenir un complexe diastéréoisomériquement enrichi constitué d'au moins 80 % d'un excès diastéréoisomérique comprenant un mélange des isomères I-RRR et I-SSS dudit complexe de gadolinium d'hexaacide de formule (I), et

**c)** Formation, à partir du complexe diastéréoisomériquement enrichi obtenu à l'étape b), du complexe de formule (II), par réaction avec le 3-amino-1,2-propanediol.

Dans la présente description, sauf mention contraire, on utilise indifféremment les expressions « Gd », « gadolinium » et « $Gd^{3+}$ » pour désigner l'ion $Gd^{3+}$. Par extension, il peut également s'agir d'une source de gadolinium libre, tels que le chlorure de gadolinium ($GdCl_3$) ou l'oxyde de gadolinium ($Gd_2O_3$).

Dans la présente invention, on désigne par « Gd libre » les formes non complexées du gadolinium, et de préférence disponibles pour une complexation. Il s'agit typiquement de l'ion $Gd^{3+}$ solubilisé dans l'eau. Par extension, il peut également s'agir d'une source de gadolinium libre, tels que le chlorure de gadolinium ($GdCl_3$) ou l'oxyde de gadolinium.

- *Etape **a)***

Au cours de cette étape a lieu une réaction de complexation entre l'hexaacide de formule (III) et le gadolinium, qui permet d'obtenir le complexe de gadolinium d'hexaacide de formule (I) telle que définie précédemment.

Selon un mode de réalisation particulier, l'étape a) comprend la réaction entre l'hexaacide de formule (III) et une source de Gd libre dans de l'eau.

Dans un mode de réalisation préféré, la source de Gd libre est du $GdCl_3$ ou du $Gd_2O_3$, de préférence du $Gd_2O_3$.

De préférence, les réactifs utilisés à l'étape a), c'est-à-dire la source de gadolinium (typiquement l'oxyde de gadolinium), l'hexaacide de formule (III) et l'eau, sont les plus purs possibles, notamment en ce qui concerne les impuretés métalliques.

Ainsi, la source de gadolinium sera avantageusement l'oxyde de gadolinium, de préférence avec une pureté supérieure à 99,99 %, et de manière encore préférée supérieure à 99,999 %.

L'eau utilisée dans le procédé comprend de préférence moins de 50 ppm de calcium, de manière encore préférée moins de 20 ppm, et de manière préférée entre toutes moins de 15 ppm de calcium. Généralement, l'eau employée dans le procédé est de l'eau désionisée, de l'eau pour injection (eau ppi) ou de l'eau purifiée.

Avantageusement, les quantités des réactifs (l'hexaacide de formule (III) et le gadolinium) utilisées lors de cette étape a) correspondent aux, ou sont proches des, proportions stœchiométriques, telles que dictées par l'équation-bilan de la réaction de complexation ayant lieu au cours de cette étape.

Par « proche des proportions stœchiométriques », on entend signifier que l'écart entre les proportions molaires dans lesquelles sont introduits les réactifs et les proportions stœchiométriques est inférieur à 15 %, notamment inférieur à 10 %, de préférence inférieur à 8 %.

Le gadolinium peut notamment être introduit en léger excès par rapport aux proportions stœchiométriques. Le rapport de la quantité de matière introduite en gadolinium sur la quantité de matière introduite en hexaacide de formule (III) est alors supérieur à 1, mais typiquement inférieur à 1,15, notamment inférieur à 1,10, avantageusement inférieur à 1,08. Autrement dit, la quantité de gadolinium introduite est supérieure à 1 équivalent (éq.), mais typiquement inférieure à 1,15 éq., notamment inférieure à 1,10 éq., avantageusement inférieure à 1,08 éq., par rapport à la quantité d'hexaacide de formule (III) introduite, qui correspond quant à elle à 1 équivalent. Dans le mode de réalisation préféré selon lequel la source de gadolinium libre est $Gd_2O_3$, la quantité de $Gd_2O_3$ introduite est alors typiquement supérieure à 0,5 éq., mais inférieure à 0,575 éq., notamment inférieure à 0,55 éq., avantageusement inférieure à 0,54 éq., par rapport à la quantité d'hexaacide de formule (III) introduite (1 éq.).

Selon un mode de réalisation particulier, l'étape a) comprend les étapes successives suivantes :

**a1)** Préparation d'une solution aqueuse d'hexaacide de formule (III), et

**a2)** Ajout, à la solution aqueuse obtenue à l'étape a1), d'une source de gadolinium libre.

Dans ce mode de réalisation, la teneur en hexaacide de formule (III) dans la solution aqueuse préparée lors de l'étape a1) est typiquement comprise entre 10% et 60%, notamment entre 15 % et 45 %, de préférence entre 20 % et 35 %, avantageusement entre 25 % et 35 %, de manière encore plus avantageuse entre 25 % et 30 % en poids par rapport au poids total de la solution aqueuse.

De manière préférentielle, les étapes a) et b) sont effectuées selon un mode de réalisation monotope (ou « one-pot » en anglais), c'est-à-dire dans le même réacteur et sans étape intermédiaire d'isolement ou de purification.

Ainsi, dans ce mode de réalisation préféré, le complexe de gadolinium d'hexaacide de formule (I) formé au cours de l'étape a) est directement soumis à l'étape b) d'isomérisation, sans être isolé ou purifié, et dans le même

réacteur que celui utilisé pour l'étape a).

- *Etape **b)***

Le complexe de gadolinium d'hexaacide de formule (I) formé par la réaction de complexation entre l'hexaacide de formule (III) et le gadolinium au cours de l'étape a) est initialement obtenu sous la forme d'un mélange de diastéréoisomères.

L'étape b) vise à enrichir le mélange de diastéréoisomères en les isomères I-RRR et I-SSS, pour obtenir le complexe de gadolinium d'hexaacide de formule (I) diastéréoisomériquement enrichi constitué d'au moins 85 %, notamment d'au moins 90 %, en particulier d'au moins 95 %, de préférence d'au moins 97 %, avantageusement d'au moins 98 %, plus avantageusement d'au moins 99 % de l'excès diastéréoisomérique comprenant le mélange des isomères I-RRR et I-SSS.

De préférence, ledit excès diastéréoisomérique est constitué d'au moins 70 %, notamment d'au moins 80 %, avantageusement d'au moins 90 %, de préférence d'au moins 95 % du mélange d'isomères I-RRR et I-SSS.

Avantageusement, ledit excès diastéréoisomérique consiste en le mélange d'isomères I-RRR et I-SSS.

Les inventeurs ont en effet découvert que des facteurs tels que le pH et la température de la solution de complexe de gadolinium d'hexaacide de formule (I) obtenue à l'issue de l'étape a) ont une influence sur le rapport dans lequel les différents isomères du complexe de formule (I) sont présents au sein du mélange de diastéréoisomères. Au cours du temps, le mélange tend à s'enrichir en un groupe d'isomères comprenant les isomères qui sont, de façon étonnante, les plus stables thermodynamiquement mais aussi chimiquement, en l'espèce les isomères I-RRR et I-SSS.

Le terme « mélange d'isomères I-RRR et I-SSS » recouvre également, par extension, le cas où seul l'un des isomères, qu'il s'agisse du I-RRR ou du I-SSS, est présent. Toutefois, le terme « mélange d'isomères I-RRR et I-SSS » désigne préférentiellement l'ensemble des cas où chacun des isomères I-RRR et I-SSS est présent en une quantité variable mais non nulle.

Dans un mode de réalisation préféré, les isomères I-RRR et I-SSS sont présents au sein dudit mélange dans un rapport compris entre 65/35 et 35/65, notamment entre 60/40 et 40/60, en particulier entre 55/45 et 45/55. Avantageusement, le mélange de d'isomères I-RRR/I-SSS est un mélange racémique (50/50).

L'étape b) d'isomérisation du complexe de gadolinium d'hexaacide de formule (I) dans une solution aqueuse est typiquement conduite à un pH compris entre 2 et 4, notamment entre 2 et 3, avantageusement entre 2,2 et 2,8.

Le pH est préférentiellement ajusté avec un acide, de préférence un acide inorganique, tel que l'acide chlorhydrique, l'acide bromhydrique, l'acide sulfurique, l'acide nitrique ou l'acide phosphorique, par exemple avec de l'acide chlorhydrique.

Il est tout à fait surprenant que dans de telles conditions de pH, un enrichissement du mélange en des isomères particuliers, en l'espèce les isomères I-RRR et I-SSS, se produise, dans la mesure où il est connu dans l'art que les chélates de gadolinium sont caractérisés par une faible inertie cinétique en milieu acide. En effet, plus la concentration en ions $H^+$ est élevée dans le milieu, plus la probabilité qu'un proton soit transféré sur l'un des atomes donneurs du ligand est forte, entrainant ainsi la dissociation du complexe. Par conséquent, l'homme du métier se serait attendu à ce que le fait de placer le complexe de gadolinium d'hexaacide de formule (I) en une solution aqueuse à un pH compris entre 2 et 4 entraîne la dissociation dudit complexe, et non pas son isomérisation en I-RRR et I-SSS.

Il est à noter que la gamme de pH recommandée par le document EP 1 931 673 pour la complexation de l'hexaacide de formule (III), à savoir 5,0 - 6,5, ne permet pas d'obtenir le complexe de formule (I) enrichi en ses isomères I-RRR et I-SSS.

L'étape b) est réalisée typiquement à une température comprise entre 80°C et 130°C, notamment entre 90°C et 125°C, de préférence entre 98°C et 122°C, avantageusement entre 100°C et 120°C, typiquement pendant une durée comprise entre 10h et 72h, notamment entre 10h et 60h, avantageusement entre 12h et 48h.

Contre toute attente, de telles conditions de température, qui, cumulées aux conditions de pH susmentionnées, devraient favoriser l'instabilité du chélate de gadolinium, n'aboutissent pas à sa décomplexation ou à la formation de toute autre impureté, mais à son isomérisation en I-RRR et I-SSS.

Dans un mode de réalisation particulier, la solution aqueuse de l'étape b) comprend de l'acide acétique. L'étape b) est alors avantageusement réalisée à une température comprise entre 100°C et 120°C, notamment entre 110°C et 118°C, typiquement pendant une durée comprise entre 12h et 48h, notamment entre 20h et 30h, en particulier entre 24h et 26h.

L'acide acétique est de préférence ajouté avant le chauffage de la solution de complexe de gadolinium d'hexaacide de formule (I) obtenue lors de l'étape a) dans une quantité telle que la teneur en acide acétique est comprise entre 25 % et 75 %, notamment entre 40 % et 50 % en masse par rapport à la masse d'hexaacide de formule (III) utilisée lors de l'étape a).

Lorsque la solution aqueuse est chauffée à une température avantageusement comprise entre 100°C et 120°C, typiquement entre 110°C et 118°C, de l'acide acétique est ajouté au fur et à mesure que l'eau s'évapore, de façon à

maintenir un volume de solution constant.

Selon un mode de réalisation préféré, à l'issue de l'étape b), le complexe diastéréoisomériquement enrichi est isolé par cristallisation, de préférence par cristallisation par ensemencement.

Dans ce mode de réalisation, l'étape b) comprend les étapes successives suivantes :

**b1)** Isomérisation par chauffage du complexe de gadolinium d'hexaacide de formule (I) dans une solution aqueuse à pH compris entre 2 et 4, pour obtenir un complexe diastéréoisomériquement enrichi constitué d'au moins 80% de l'excès diastéréoisomérique comprenant le mélange des isomères I-RRR et I-SSS dudit complexe de gadolinium d'hexaacide de formule (I), et

**b2)** Isolement par cristallisation dudit complexe diastéréoisomériquement enrichi de préférence par cristallisation par ensemencement.

L'étape b2) de cristallisation vise d'une part à éliminer les impuretés éventuellement présentes dans la solution aqueuse, qui peuvent résulter d'étapes antérieures, de façon à obtenir un produit de plus grande pureté, décoloré, sous forme de cristaux, et d'autre part à poursuivre l'enrichissement diastéréoisomérique du complexe de gadolinium d'hexaacide de formule (I), de façon à obtenir un excès diastéréoisomérique comprenant le mélange des isomères I-RRR et I-SSS dudit complexe supérieur à celui obtenu à l'issue de l'étape b1).

En effet, les isomères I-RRR et I-SSS du complexe hexaacide de formule (I) cristallisent dans l'eau. En revanche, le complexe de gadolinium d'hexaacide de formule (I) non enrichi en lesdits isomères ne cristallise pas.

Le fait que les isomères I-RRR et I-SSS, en lesquels le complexe tend à s'enrichir au cours de l'étape b) (et ce contre toute attente, au vu des conditions dans lesquelles elle est réalisée), sont les seuls isomères du complexe à cristalliser dans l'eau constitue un résultat tout à fait inattendu. L'isomérisation et la cristallisation contribuent ainsi de manière synergique à l'enrichissement en isomères I-RRR et I-SSS, et dès lors à l'efficacité globale du procédé selon l'invention.

Par ailleurs, il est à noter que la cristallisation dans l'eau des isomères d'intérêt du complexe de gadolinium d'hexaacide de formule (I) permet d'éviter un ajout de solvant tel que décrit dans l'exemple 7 du document EP 1 931 673, qui met en jeu une étape de précipitation dans l'éthanol du sel trisodique dudit complexe.

L'étape b2) est réalisée avantageusement à une température comprise entre 10°C et 70°C, notamment entre 30°C et 65°C, en particulier entre 35° et 60°C.

Selon une variante, après abaissement de la température de la solution aqueuse, de façon à ce que celle-ci soit comprise dans les gammes indiquées ci-dessus, le processus de cristallisation est induit par ensemencement. La « cristallisation par ensemencement », aussi appelée « cristallisation par amorçage », comprend l'introduction dans le réacteur dans lequel la cristallisation est effectuée (aussi appelé cristallisoir) d'une quantité connue de cristaux, appelée « semence » ou « amorce ». Cela permet de diminuer le temps de cristallisation. La cristallisation par ensemencement est bien connue de l'homme du métier.

Dans le procédé selon l'invention, l'ensemencement par utilisation d'une amorce, en l'espèce des cristaux de complexe de gadolinium d'hexaacide de formule (I) diastéréoisomériquement enrichi ajoutés dans la solution aqueuse du complexe diastéréoisomériquement enrichi dont la température a été préalablement abaissée, permet d'obtenir une nucléation, et ainsi d'initier la cristallisation. La durée de la cristallisation par ensemencement est avantageusement comprise entre 2h et 20h, de préférence entre 6h et 18h, typiquement, elle est de 16h.

Les cristaux de complexe de gadolinium d'hexaacide de formule (I) diastéréoisomériquement enrichi sont alors isolés typiquement par filtration et séchage, en mettant en œuvre toute technique bien connue de l'homme de l'art.

Avantageusement, le degré de pureté du complexe de gadolinium d'hexaacide de formule (I) diastéréoisomériquement enrichi isolé à l'issue de l'étape b2) est supérieur à 95 %, notamment supérieur à 98 %, avantageusement supérieur à 99 %, ledit degré de pureté étant exprimé en pourcentage massique du complexe de formule (I) par rapport à la masse totale obtenue à l'issue de l'étape b2).

Dans un mode de réalisation particulier, le complexe diastéréoisomériquement enrichi de l'étape b) isolé par cristallisation est à nouveau purifié par recristallisation, pour obtenir un complexe diastéréoisomériquement enrichi et purifié.

Dans ce mode de réalisation, l'étape b) comprend, outre les étapes successives b1) et b2) précédemment décrites, une étape **b3)** de purification par recristallisation du complexe de gadolinium d'hexaacide de formule (I) diastéréoisomériquement enrichi isolé.

L'étape b3) de recristallisation vise, à l'instar de l'étape b2) de cristallisation, d'une part, à obtenir un produit de plus grande pureté, et, d'autre part, à poursuivre l'enrichissement diastéréoisomérique du complexe de gadolinium d'hexaacide de formule (I), de façon à obtenir un excès diastéréoisomérique comprenant le mélange des isomères I-RRR et I-SSS dudit complexe supérieur à celui obtenu à l'issue de l'étape b2).

L'étape b3) comprend typiquement les sous-étapes successives suivantes :

- mise en suspension du complexe de gadolinium d'hexaacide de formule (I) diastéréoisomériquement enrichi isolé lors de l'étape b2) en solution aqueuse, de préférence dans de l'eau,
- solubilisation dudit complexe par chauffage à une température avantageusement comprise entre 80°C et 120°C, par exemple à 100°C,
- recristallisation, de préférence par ensemencement, à une température avantageusement comprise entre 10°C et 90°C, notamment entre 20°C et 87°C, en particulier entre 55°C et 85°C, typiquement pendant une durée comprise entre 2h et 20h, notamment entre 6h et 18h, et
- isolement des cristaux de complexe de gadolinium d'hexaacide de formule (I) diastéréoisomériquement enrichi et purifié, par exemple par filtration et séchage.

Le degré de pureté du complexe de gadolinium d'hexaacide de formule (I) diastéréoisomériquement enrichi purifié isolé à l'issue de l'étape b3) est typiquement supérieur à 98 %, notamment supérieur à 99 %, avantageusement supérieur à 99,5 %, ledit degré de pureté étant exprimé en pourcentage massique du complexe de formule (I) par rapport à la masse totale obtenue à l'issue de l'étape b2).

Dans un autre mode de réalisation, le complexe diastéréoisomériquement enrichi de l'étape b) est encore enrichi par décomplexation sélective des diastéréoisomères du complexe de formule (I) autres que les diastéréoisomères I-RRR et I-SSS, i.e. par décomplexation sélective des diastéréoisomères I-RSS, I-SRR, I-RSR, I-SRS, I-RRS et I-SSR.

Dans ce mode de réalisation, l'étape b) comprend, outre les étapes successives b1) et b2) précédemment décrites, une étape **b4)** de décomplexation sélective des diastéréoisomères du complexe de formule (I) autres que les diastéréoisomères I-RRR et I-SSS. Dans cette variante, l'étape b) peut en outre comprendre l'étape b3) précédemment décrite, ladite étape b3) étant mise en œuvre entre les étapes b2) et b4), ou après l'étape b4).

L'étape b4) de décomplexation sélective vise à poursuivre l'enrichissement diastéréoisomérique du complexe de gadolinium d'hexaacide de formule (I), de façon à obtenir un excès diastéréoisomérique comprenant le mélange des isomères I-RRR et I-SSS dudit complexe supérieur à celui obtenu à l'issue de l'étape b2) ou à l'issue de l'étape b3), lorsque celle-ci est mise en œuvre préalablement à l'étape b4).

L'étape b4) comprend typiquement les sous-étapes successives suivantes :

- mise en suspension du complexe de gadolinium d'hexaacide de formule (I) diastéréoisomériquement enrichi isolé lors de l'étape b2) ou lors de l'étape b3) dans de l'eau,
- ajout d'une base, par exemple de soude,
- chauffage à une température avantageusement comprise entre 30°C et 60°C, notamment entre 35°C et 55°C, par exemple à 40°C, typiquement pendant une durée comprise entre 2h et 20h, notamment entre 10h et 18h,
- refroidissement à une température avantageusement comprise entre 10°C et 30°C, par exemple à 30°C, et
- isolement du complexe de gadolinium d'hexaacide de formule (I) diastéréoisomériquement enrichi et purifié, par exemple par filtration et séchage.

L'étape b4) est rendue possible par le fait que les isomères I-RRR et I-SSS sont les plus stables en milieu basique. De telles conditions basiques favorisent la formation d'hydroxyde de gadolinium, et par conséquent la décomplexation des isomères les moins stables.

Ainsi, il convient de noter que, de manière surprenante, les isomères I-RRR et I-SSS sont plus stables à la fois en milieu acide, ce qui permet l'étape b1) d'isomérisation, et en milieu basique, ce qui permet l'étape b4) de décomplexation sélective.

Dans un mode de réalisation préféré, le complexe diastéréoisomériquement enrichi obtenu à l'issue de l'étape b) selon l'une quelconque des variantes décrites ci-dessus présente au moins 85 %, notamment au moins 90 %, en particulier au moins 95 %, de préférence au moins 97 %, avantageusement au moins 98 %, plus avantageusement au moins 99 % de l'excès diastéréoisomérique comprenant le mélange des isomères I-RRR et I-SSS.

De préférence, ledit excès diastéréoisomérique est constitué d'au moins 70 %, notamment d'au moins 80 %, avantageusement d'au moins 90 %, de préférence d'au moins 95 % du mélange d'isomères I-RRR et I-SSS.

Avantageusement, ledit excès diastéréoisomérique consiste en le mélange d'isomères I-RRR et I-SSS.

Le terme « mélange d'isomères I-RRR et I-SSS » recouvre également, par extension, le cas où seul l'un des isomères, qu'il s'agisse du I-RRR ou du I-SSS, est présent. Toutefois, le terme « mélange d'isomères I-RRR et I-SSS » désigne préférentiellement l'ensemble des cas où chacun des isomères I-RRR et I-SSS est présent en une quantité variable mais non nulle.

Dans un mode de réalisation préféré, les isomères I-RRR et I-SSS sont présents au sein dudit mélange dans un rapport compris entre 65/35 et 35/65, notamment entre 60/40 et 40/60, en particulier entre 55/45 et 45/55. Avantageusement, le mélange de d'isomères I-RRR/I-SSS est un mélange racémique (50/50).

- *Etape **c)***

L'étape c) vise à former le complexe de formule (II) à partir de son précurseur, le complexe de gadolinium d'hexaacide de formule (I) diastéréoisomériquement enrichi obtenu lors de l'étape b).

Au cours de cette étape, les trois fonctions acides carboxyliques du complexe hexaacide de formule (I) portées par les atomes de carbone situés en position $\gamma$ sur les chaînes latérales du complexe, par rapport aux atomes d'azote du macrocycle sur lesquels sont greffées lesdites chaînes latérales, sont converties en fonctions amide, par réaction d'amidification avec le 3-amino-1,2-propanediol, sous forme racémique ou énantiomériquement pure, de préférence sous forme racémique.

Cette réaction d'amidification ne modifie pas la configuration absolue des trois atomes de carbones asymétriques situés en position $\alpha$ sur les chaînes latérales, par rapport aux atomes d'azote du macrocycle sur lesquels sont greffées lesdites chaînes latérales. Par conséquent, l'étape c) permet d'obtenir le complexe de formule (II) avec un excès diastéréoisomérique comprenant un mélange des isomères II-RRR et II-SSS identique à l'excès diastéréoisomérique comprenant un mélange des isomères I-RRR et I-SSS avec lequel est obtenu le complexe de gadolinium d'hexaacide de formule (I) diastéréoisomériquement enrichi obtenu à l'issue de l'étape b), qui est d'au moins 80 %.

Dans un mode de réalisation préféré, le complexe de formule (II) obtenu à l'issue de l'étape c) présente au moins 85 %, notamment au moins 90 %, en particulier au moins 92 %, de préférence au moins 94 %, avantageusement au moins 97 %, plus avantageusement au moins 99 % de l'excès diastéréoisomérique comprenant le mélange d'isomères II-RRR et II-SSS.

De préférence, ledit excès diastéréoisomérique est constitué d'au moins 70 %, notamment d'au moins 80 %, avantageusement d'au moins 90 %, de préférence d'au moins 95 % du mélange d'isomères II-RRR et II-SSS.

Avantageusement, ledit excès diastéréoisomérique consiste en le mélange d'isomères II-RRR et II-SSS.

Le terme « mélange d'isomères II-RRR et II-SSS » recouvre également, par extension, le cas où seul l'un des isomères, qu'il s'agisse du II-RRR ou du II-SSS, est présent. Toutefois, le terme « mélange d'isomères II-RRR et II-SSS » désigne préférentiellement l'ensemble des cas où chacun des isomères II-RRR et II-SSS est présent en une quantité variable mais non nulle.

Dans un mode de réalisation préféré, les isomères II-RRR et II-SSS sont présents au sein dudit mélange dans un rapport compris entre 65/35 et 35/65, notamment entre 60/40 et 40/60, en particulier entre 55/45 et 45/55. Avantageusement, les isomères II-RRR et II-SSS sont présents au sein du mélange dans un rapport 50/50.

La réaction d'amidification peut être effectuée selon toutes les méthodes bien connues de l'homme du métier, notamment en présence d'un agent activant des fonctions acides carboxyliques et/ou en catalyse acide.

Elle peut notamment être réalisée selon les méthodes décrites dans le brevet EP 1 931 673, notamment au paragraphe [0027] de ce brevet.

Dans un mode de réalisation particulier, l'étape c) comprend l'activation des fonctions acides carboxyliques (-COOH) du complexe hexaacide de formule (I) portées par les atomes de carbone situés en position $\gamma$ sur les chaînes latérales du complexe, par rapport aux atomes d'azote du macrocycle sur lesquels sont greffées lesdites chaînes latérales, sous la forme de fonctions dérivées comportant un groupement carbonyle (C=O), qui sont telles que l'atome de carbone du groupement carbonyle est plus électrophile que l'atome de carbone du groupement carbonyle des fonctions acides carboxyliques. Ainsi, selon ce mode de réalisation particulier, lesdites fonctions acides carboxyliques peuvent notamment être activées sous la forme de fonctions esters, de chlorures d'acyle, d'anhydrides d'acide, ou sous toute forme activée susceptible de conduire à une liaison amide. Les formes activées susceptibles de conduire à une liaison amide sont bien connues de l'homme du métier et peuvent être par exemple obtenues par l'ensemble des méthodes connues en chimie peptidique pour créer une liaison peptidique. Des exemples de telles méthodes sont données dans la publication Synthesis of peptides and peptidomimetics vol.E22a, p425-588, Houben-Weyl et al., Goodman Editor, Thieme-Stuttgart-New York (2004), et, parmi ces exemples, peuvent être notamment citées les méthodes d'activation des acides carboxyliques via un azoture (azoture d'acyle), par exemple par l'action d'un réactif tel que l'azoture de diphénylphosphoryle (communément désigné par l'acronyme anglais DPPA), l'utilisation des carbodiimides seules ou en présence de catalyseurs (par exemple le N-hydroxysuccinimide et ses dérivés), l'utilisation d'un carbonyldiimidazole (1,1'-carbonyldiimidazole, CDI), l'utilisation des sels de phosphonium comme l'hexafluorophosphate de benzotriazol-1-yloxy-tris(dimethylamino)phosphonium (communément désigné par l'acronyme anglais BOP) ou encore les uroniums comme l'hexafluorophosphate de 2-(1H-benzotriazol-1-yl)-1,1,3,3-tétraméthyluronium (communément désigné par l'acronyme anglais HBTU).

De préférence, l'étape c) comprend l'activation des fonctions acides carboxyliques (-COOH) susmentionnées sous la forme de fonctions esters, chlorures d'acyle ou anhydrides d'acide.

Ce mode de réalisation est préféré au couplage peptidique par activation de la fonction acide carboxylique à l'aide d'un agent de couplage tel que EDCI/HOBT comme décrit dans le document EP 1 931 673. En effet, un tel couplage entraîne la formation d'un équivalent de 1-éthyl-3-[3-(diméthylamino)propyl]urée, qui doit être éliminé, notamment par chromatographie sur silice ou par extraction liquide/liquide en ajoutant un solvant. Indépendamment de la complexification

du procédé engendrée par une telle étape additionnelle, la mise en œuvre de telles méthodes de purification n'est pas souhaitable, comme discuté précédemment. De plus, l'usage de HOBT est en soit problématique, en cela qu'il s'agit d'un produit explosif.

Par« fonction ester », on entend désigner au sens de la présente invention un groupement -C(O)O-. Il peut s'agir en particulier d'un groupement -C(O)O-$R_1$, dans lequel $R_1$ correspond à un groupement ($C_1$-$C_6$)alkyle.

Par groupement « ($C_1$-$C_6$)alkyle », on entend, au sens de la présente invention, une chaîne hydrocarbonée saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par « fonction chlorure d'acyle », également appelée « fonction chlorure d'acide », on entend désigner au sens de la présente invention un groupement -CO-Cl.

Par « fonction anhydride d'acide », on entend désigner au sens de la présente invention un groupement -CO-O-CO-. Il peut s'agir en particulier d'un groupement -CO-O-CO-$R_2$, dans lequel $R_2$ correspond à un groupement ($C_1$-$C_6$)alkyle.

Les réactions de conversion d'une fonction acide carboxylique en fonction ester, chlorure d'acyle ou anhydride d'acide sont bien connues de l'homme du métier, qui pourra les mettre en œuvre selon toute méthode usuelle dont il est familier.

Le complexe de formule (II) est ensuite obtenu par aminolyse des fonctions acides carboxyliques activées sous la forme de fonctions esters, chlorures d'acyle ou anhydrides d'acide, notamment esters ou anhydrides d'acide, de préférence esters, par réaction avec le 3-amino-1,2-propanediol, sous forme racémique ou énantiomériquement pure, de préférence sous forme racémique.

De manière préférentielle, les étapes d'activation des fonctions acides carboxyliques et d'aminolyse sont effectuées selon un mode de réalisation monotope (ou « one-pot » en anglais), c'est-à-dire dans le même réacteur et sans étape intermédiaire d'isolement ou de purification de l'intermédiaire comportant les fonctions acides carboxyliques activées sous la forme de fonctions esters, chlorures d'acyle ou anhydrides d'acide, notamment esters ou anhydrides d'acide, de préférence esters.

Selon un mode de réalisation particulier, l'étape c) comprend les étapes successives suivantes :

**c1)** formation d'un complexe activé de formule (VII),

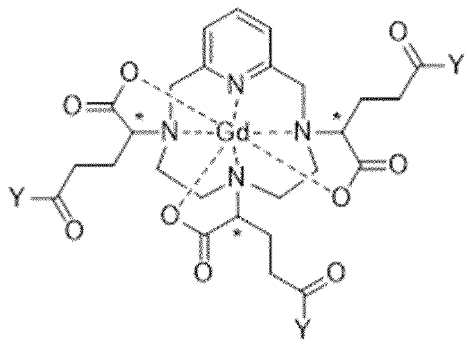

(VII)

dans laquelle Y représente un atome de chlore, un groupement -O$R_1$ ou -O-C(O)-$R_2$, de préférence Y représente un groupement -O$R_1$ ou -O-C(O)-$R_2$, avec $R_1$ et $R_2$ correspondant, indépendamment l'un de l'autre, à un groupe ($C_1$-$C_6$)alkyle, et

**c2)** aminolyse du complexe activé de formule (VII) avec le 3-amino-1,2-propanediol.

Comme cela apparaîtra clairement à l'homme du métier, la réaction de formation du complexe activé de formule (VII) ne modifie pas la configuration absolue des trois atomes de carbones asymétriques situés en position $\alpha$ sur les chaînes latérales, par rapport aux atomes d'azote du macrocycle sur lesquels sont greffées lesdites chaînes latérales. Par conséquent, l'étape c1) permet d'obtenir le complexe activé de formule (VII) avec un excès diastéréoisomérique comprenant un mélange des isomères VII-RRR et VII-SSS, de formules (VII-RRR) et (VII-SSS) représentées ci-après, identique à l'excès diastéréoisomérique comprenant un mélange des isomères I-RRR et I-SSS avec lequel est obtenu le complexe de gadolinium d'hexaacide de formule (I) diastéréoisomériquement enrichi obtenu à l'issue de l'étape b), qui est d'au moins 80 %.

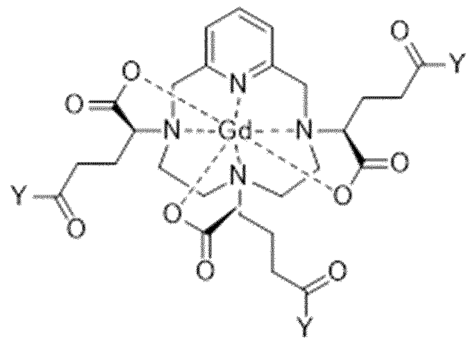

(VII-SSS)

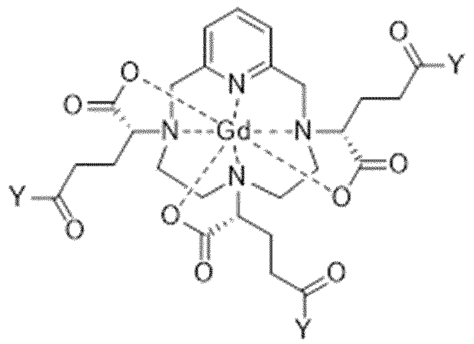

(VII-RRR)

Dans le cas où Y représente un atome de chlore, l'étape c1) est typiquement réalisée par réaction entre le complexe de gadolinium d'hexaacide de formule (I) diastéréoisomériquement enrichi obtenu lors de l'étape b) et le chlorure de thionyle ($SOCl_2$).

Dans le cas où Y représente un groupement -O-C(O)-$CH_3$, l'étape c1) est typiquement réalisée par réaction entre le complexe de gadolinium d'hexaacide de formule (I) diastéréoisomériquement enrichi obtenu lors de l'étape b) et le chlorure d'acétyle.

Dans un mode de réalisation avantageux, l'étape c) comprend l'activation des fonctions acides carboxyliques (-COOH) susmentionnées sous la forme de fonctions esters.

Selon ce mode de réalisation, l'étape c) peut plus particulièrement comprendre les étapes successives suivantes :

**c1)** formation d'un triester de formule (VIII),

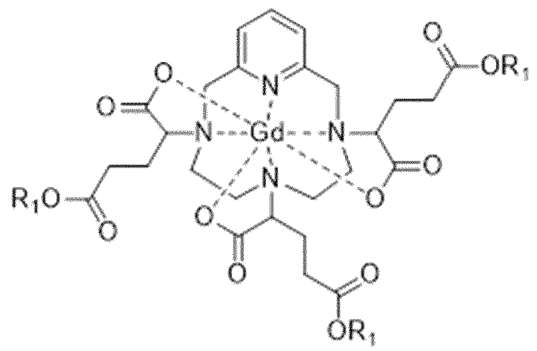

(VIII)

dans laquelle $R_1$ représente un groupe ($C_1$-$C_6$)alkyle, et

**c2)** aminolyse du triester de formule (VIII) avec le 3-amino-1,2-propanediol.

L'étape c1) est typiquement réalisée dans l'alcool de formule $R_1OH$, qui joue à la fois le rôle de solvant et de réactif, en présence d'un acide tel que l'acide chlorhydrique.

Dans un premier temps, le complexe de gadolinium d'hexaacide de formule (I) et l'alcool $R_1OH$ sont chargés dans le réacteur. Le milieu réactionnel est ensuite refroidi à une température inférieure à 10°C, notamment inférieure à 5 °C, typiquement à 0°C, et une solution acide de l'alcool $R_1OH$, typiquement d'acide chlorhydrique dans $R_1OH$ est alors progressivement ajoutée. Le milieu réactionnel est maintenu sous agitation à température ambiante (c'est-à-dire à une température entre 20 et 25°C) pendant une durée typiquement supérieure à 5h, de préférence comprise entre 10h et 20h. Le milieu réactionnel est refroidi à une température inférieure à 10°C, notamment comprise entre 0°C et 5 °C, préalablement à l'étape c2).

L'étape c2) est également typiquement réalisée dans l'alcool de formule $R_1OH$, en présence d'un acide tel que l'acide chlorhydrique.

Ainsi, les étapes c1) et c2) peuvent être aisément mises en œuvre selon un mode de réalisation monotope (ou « one-pot » en anglais). Avantageusement, le triester de formule (VII) n'est pas isolé entre les étapes c1) et c2).

Toutefois, afin de favoriser la réaction d'aminolyse, au cours de l'étape c2), l'alcool de formule $R_1OH$ est de préférence éliminé par distillation sous vide.

Par « distillation sous vide », on entend, au sens de la présente invention, la distillation d'un mélange réalisée à une pression comprise entre 10 et 500 mbar, notamment entre 10 et 350 mbar, de préférence entre 10 et 150 mbar, en particulier entre 50 et 100 mbar.

De même, afin de favoriser la réaction d'aminolyse, lors de l'étape c2), le 3-amino-1,2-propanediol est introduit en large excès. Typiquement, la quantité de matière de 3-amino-1,2-propanediol introduite est supérieure à 4 éq., notamment supérieure 7 éq., avantageusement supérieure à 10 éq., par rapport à la quantité de matière de complexe de gadolinium d'hexaacide de formule (I) diastéréoisomériquement enrichi initialement introduite au cours de l'étape c), qui correspond quant à elle à 1 équivalent.

De manière surprenante, en dépit des conditions acides typiquement employées au cours des étapes c1) et c2), qui devraient augmenter l'instabilité cinétique des complexes de gadolinium, on n'observe pas de décomplexation ou d'isomérisation du triester de formule (VIII). Le triamide désiré est obtenu avec un très bon taux de conversion et la configuration absolue des trois atomes de carbones asymétriques situés en position $\alpha$ sur les chaînes latérales, par rapport aux atomes d'azote du macrocycle, est conservée.

Par ailleurs, il est à noter que, de manière générale, les réactions d'amidification par réaction directe entre un ester et une amine sont très peu décrites dans la littérature (voir à ce sujet K. C. Nadimpally et al., Tetrahedron Letters, 2011, 52, 2579-2582).

Dans un mode de réalisation préféré, l'étape c) comprend les étapes successives suivantes :

**c1)** formation d'un triester de méthyle de formule (IV),

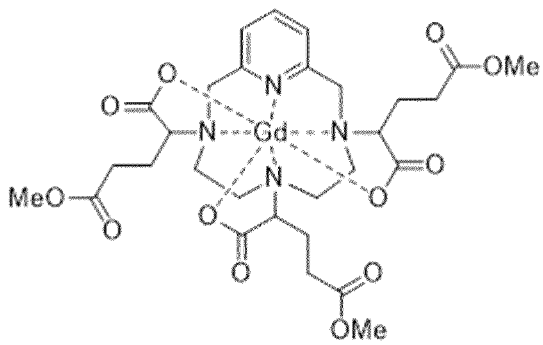

(IV)

notamment par réaction dans du méthanol en présence d'un acide tel que l'acide chlorhydrique, et

**c2)** aminolyse du triester de méthyle de formule (IV) avec le 3-amino-1,2-propanediol, notamment dans du méthanol en présence d'un acide tel que l'acide chlorhydrique.

Avantageusement, le triester de méthyle de formule (IV) n'est pas isolé entre les étapes c1) et c2).

Dans un mode de réalisation préféré, au cours de l'étape c2), le méthanol est éliminé par distillation sous vide,

jusqu'à atteindre une température typiquement supérieure à 55°C, notamment comprise entre 60°C et 65°C, et le milieu réactionnel est maintenu à cette température sous vide pendant une durée typiquement supérieure à 5h, notamment comprise entre 10h et 20h, avant d'être refroidi à température ambiante et dilué avec de l'eau.

La présente invention englobe toutes les combinaisons des modes de réalisations particuliers, avantageux ou préférés décrits ci-dessus en lien avec chaque étape du procédé.

Dans un mode de réalisation préféré, le procédé selon l'invention implique donc un complexe de gadolinium de triester de formule (VIII) :

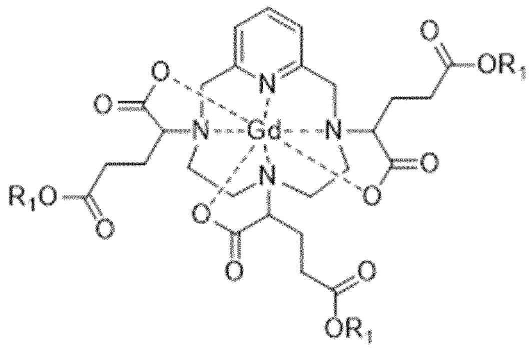

(VIII)

constitué d'au moins 80 % d'un excès diastéréoisomérique comprenant un mélange d'isomères VIII-RRR et VIII-SSS de formules :

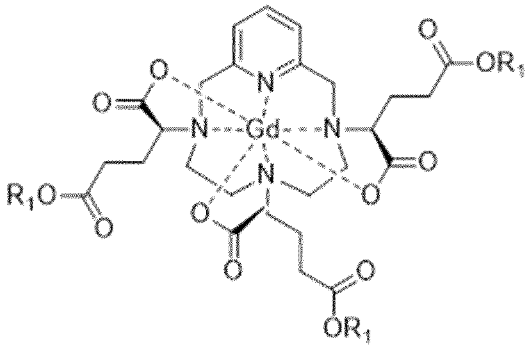

(VIII-SSS)

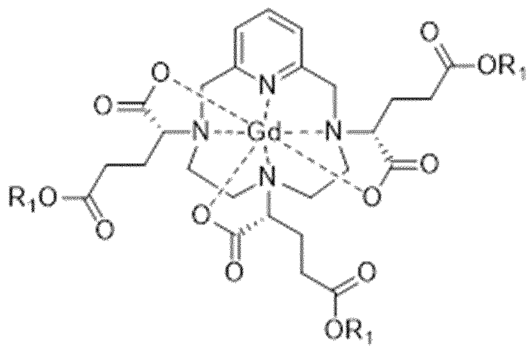

(VIII-RRR)

Par « excès diastéréoisomérique » on entend désigner, dans le cadre de la présente invention, et en ce qui concerne le complexe de gadolinium de triester de formule (VIII), le fait que ledit complexe est majoritairement présent sous la forme d'un isomère ou groupe d'isomères choisi(s) parmi les diastéréoisomères VIII-RRR, VIII-SSS, VIII-RRS,

VIII-SSR, VIII-RSS, VIII-SRR, VIII-RSR et VIII-SRS. Ledit excès diastéréoisomérique est exprimé en pourcentage, et correspond à la quantité que représente l'isomère ou le groupe d'isomères majoritaire par rapport à la quantité totale du complexe de triester de formule (VIII). Il est entendu que ce pourcentage peut être aussi bien molaire que massique, dans la mesure où des isomères ont, par définition, la même masse molaire.

Dans un mode de réalisation particulier du procédé selon l'invention, le complexe de gadolinium de triester de formule (VIII) selon l'invention présente au moins 85%, notamment au moins 90 %, en particulier au moins 95 %, de préférence au moins 97 %, avantageusement au moins 98 %, plus avantageusement au moins 99 % de l'excès diastéréoisomérique comprenant le mélange d'isomères VIII-RRR et VIII-SSS.

De préférence, ledit excès diastéréoisomérique est constitué d'au moins 70 %, notamment d'au moins 80 %, avantageusement d'au moins 90 %, de préférence d'au moins 95 % du mélange d'isomères VIII-RRR et VIII-SSS.

Avantageusement, ledit excès diastéréoisomérique consiste en le mélange d'isomères VIII-RRR et VIII-SSS.

Le terme « mélange d'isomères VIII-RRR et VIII-SSS » recouvre également le cas où seul l'un des isomères, qu'il s'agisse du VIII-RRR ou du VIII-SSS, est présent. Toutefois, le terme « mélange d'isomères VIII-RRR et VIII-SSS » désigne préférentiellement l'ensemble des cas où chacun des isomères VIII-RRR et VIII-SSS est présent en une quantité variable mais non nulle.

Dans un mode de réalisation préféré du procédé selon l'invention, les isomères VIII-RRR et VIII-SSS sont présents au sein dudit mélange dans un rapport compris entre 65/35 et 35/65, notamment entre 60/40 et 40/60, en particulier entre 55/45 et 45/55. Avantageusement, le mélange de d'isomères VIII-RRR/VIII-SSS est un mélange racémique (50/50).

Dans un mode de réalisation préféré du procédé selon l'invention, le complexe de gadolinium de triester de formule (VIII) est un complexe de gadolinium de triméthyle, c'est-à-dire complexe de gadolinium de triester de formule (VIII) dans laquelle $R_1$ est un groupement méthyle ($CH_3$).

- *Préparation de l'hexaacide de formule (III)*

L'hexaacide de formule (III), qui intervient lors de l'étape a) du procédé de préparation du complexe de formule (II) selon l'invention, peut être préparé selon toutes les méthodes d'ores et déjà connues et notamment selon les méthodes décrites dans le brevet EP 1 931 673.

Toutefois, selon un mode de réalisation préféré, l'hexaacide de formule (III) est obtenu par alkylation du pyclène de formule (V) :

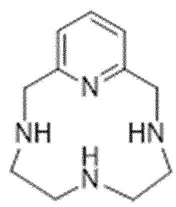

(V)

avec un composé de formule $R_3OOC\text{-}CHG_p\text{-}(CH_2)_2\text{-}COOR_4$ (IX), dans laquelle :

- $R_3$ et $R_4$ représentent, indépendamment l'un de l'autre, un groupe ($C_3$-$C_6$)alkyle, notamment un groupe ($C_4$-$C_6$)alkyle tel qu'un groupement butyle, isobutyle, sec-butyle, tert-butyle, pentyle ou encore hexyle, et
- $G_p$ représente un groupe partant tel qu'un groupement tosylate, triflate, ou un atome d'halogène, de préférence un atome de brome,

pour obtenir l'hexaester de formule (X)

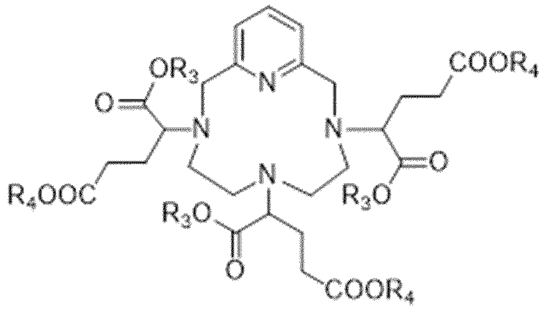

(X)

suivie d'une étape d'hydrolyse, conduisant audit hexaacide de formule (III).

Dans un mode de réalisation préféré, $R_3$ et $R_4$ sont identiques.

Selon un mode de réalisation avantageux, l'hexaacide de formule (III) est obtenu par alkylation du pyclène de formule (V) :

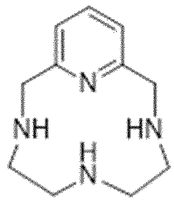

(V)

avec le 2-bromoglutarate de dibutyle, pour obtenir l'hexaester de butyle de formule (VI) :

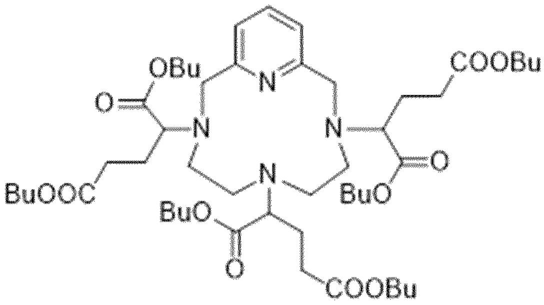

(VI)

suivie d'une étape d'hydrolyse, conduisant audit hexaacide de formule (III).

Le 2-bromoglutarate de dibutyle utilisé est sous forme racémique ou énantiomériquement pure, de préférence sous forme racémique.

L'utilisation du 2-bromoglutarate de dibutyle est particulièrement avantageuse, en comparaison à celle du 2-bromoglutarate d'éthyle décrite dans le document EP 1 931 673. En effet, le 2-bromoglutarate de diéthyle commercial est un composé relativement instable, qui se dégrade dans le temps et sous l'effet de la température. Plus précisément, cet ester a tendance à s'hydrolyser ou cycliser et donc à perdre son atome de brome. Les tentatives de purification du 2-bromoglutarate de diéthyle commercial, ou de mise au point de nouvelles voies de synthèse permettant de l'obtenir avec une pureté améliorée, et ainsi empêcher sa dégradation, n'ont pas abouti.

La réaction d'alkylation est typiquement réalisée dans un solvant polaire, de préférence dans l'eau, en particulier dans de l'eau désionisée, avantageusement en présence d'une base telle que le carbonate de potassium ou de sodium.

L'utilisation d'eau est préférée notamment à celle d'acétonitrile, décrite dans le document EP 1 931 673, pour des raisons évidentes.

La réaction est avantageusement conduite à une température comprise entre 40°C et 80°C, typiquement entre 50°C et 70°C, notamment entre 55°C et 60°C, pendant une durée comprise entre 5h et 20h, en particulier entre 8h et 15h.

L'étape d'hydrolyse est réalisée avantageusement en présence d'un acide ou d'une base, avantageusement d'une base telle que la soude. Le solvant d'hydrolyse peut être de l'eau, un alcool tel que l'éthanol, ou un mélange eau/alcool. Cette étape est conduite avantageusement à une température comprise entre 40°C et 80°C, typiquement entre 40°C et 70°C, notamment entre 50°C et 60°C, typiquement pendant une durée comprise entre 10h et 30h, en particulier entre 15h et 25h.

Dans un mode de réalisation préféré, le procédé selon l'invention implique donc l'hexaester de butyle de formule (VI) :

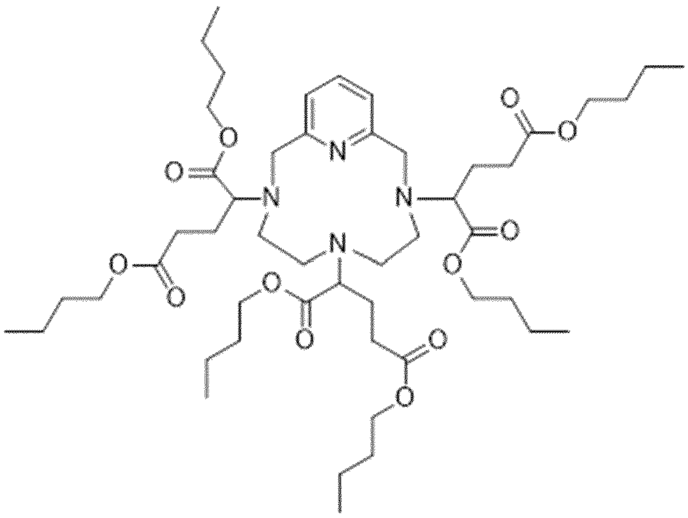

(VI)

En effet, cet hexaester se distingue par une stabilité nettement améliorée par rapport aux esters ayant une chaîne alkyle plus courte, notamment par rapport à l'hexaester d'éthyle décrit dans le document EP 1 931 673.

FIGURES :

Figure 1 : dégradation en conditions basiques des groupes d'isomères iso1 à iso4 du complexe de formule (II), exprimée en pourcentage surfacique d'un groupe d'isomère donné au cours du temps.

**EXEMPLES**

Les exemples figurant ci-après sont présentés à titre illustratif et non limitatif de l'invention.

**<u>Séparation des groupes d'isomères isoA, isoB, isoC et isoD du complexe de gadolinium d'hexaacide de formule (I) par HPLC</u>**

Un appareil HPLC constitué d'un système de pompage, d'un injecteur, d'une colonne chromatographique, d'un détecteur spectrophotométrique UV et d'une station de pilotage et de traitement des données est utilisé. La colonne chromatographique utilisée est une colonne C18 - 250 x 4,6 mm - 5 µm (gamme Symmetry®, de Waters).

- Phase mobile :

Voie A : 100% acétonitrile et Voie B : solution aqueuse d'H2SO4 (96 %) à 0,1 % v/v

- Préparation des solutions essais :

Solution du complexe de gadolinium d'hexaacide de formule (I) à 10 mg/mL dans l'eau purifiée.
- Conditions d'analyse :

| | |
|---|---|
| Température colonne | 25°C |
| Température échantillon | Température ambiante (20-25°C) |
| Débit | 1,0 ml/min |
| Volume d'injection | 20 µl |
| Détection UV | 200 nm |
| Durée d'analyse | 60 min |

- Gradient :

| Temps | % Acn | % $H_2SO_4$ 0,1 % |
|---|---|---|
| 0 | 1 | 99 |
| 10 | 5 | 95 |
| 40 | 10 | 90 |
| 50 | 25 | 75 |
| 55 | 1 | 99 |
| 60 | 1 | 99 |
| %Acn : % v/v d'acétonitrile dans la phase mobile<br>% $H_2SO_4$ 0,1 % : % v/v de la solution de $H_2SO_4$ à 0,1% v/v dans la phase mobile | | |

4 pics principaux sont obtenus. Le pic 4 de la trace HPLC, à savoir l'isoD, correspond à un temps de rétention de 35,7 minutes.

**Séparation des groupes d'isomères isoA, isoB, isoC et isoD du complexe de gadolinium d'hexaacide de formule (I) par UHPLC**

Un appareil UHPLC constitué d'un système de pompage, d'un injecteur, d'une colonne chromatographique, d'un détecteur UV et d'une station de données est utilisé. La colonne chromatographique utilisée est une colonne UHPLC 150 x 2,1 mm -1,8 µm (colonne ACQUITY UPLC HSS T3 de Waters). Il s'agit d'une colonne UPLC en phase inverse à particules sphériques constituées de silice avec un greffage C18 (octadécyl) trifonctionnel, et dont les silanols ont été traités par des agents de coiffage (end-capped). Elle est en outre caractérisée par une longueur de 150 mm, un diamètre intérieur de 2,1 mm, une granulométrie de 1,8 µm, une porosité de 100 Å et un taux de carbone de 11%.

De manière préférentielle, la phase stationnaire utilisée doit être compatible avec les phases mobiles aqueuses.

- Phase mobile :

Voie A: 100% acétonitrile et Voie B : solution aqueuse d'$H_2SO_4$ (96 %) à 0,1 % v/v

- Préparation des solutions essais :

Solution du complexe de gadolinium d'hexaacide de formule (I) à 0,8 mg/mL dans de l'eau purifiée

- Conditions d'analyse :

| | |
|---|---|
| Température colonne | 35°C |
| Température échantillon | Température ambiante (20-25°C) |
| Débit | 0,4 mL/min |
| Volume d'injection | 10 µl |
| Détection UV | 200 nm |
| Durée d'analyse | 32 min |

- Gradient :

| Temps | % Acn | % $H_2SO_4$ 0,1 % |
|---|---|---|
| 0 | 1 | 99 |
| 14 | 8 | 92 |
| 20 | 11 | 89 |
| 25 | 25 | 75 |
| 27 | 1 | 99 |
| 32 | 1 | 99 |

4 pics principaux sont obtenus. Le pic 4 de la trace UHPLC, à savoir l'isoD, correspond à un temps de rétention de 17,4 minutes.

**Séparation des groupes d'isomères iso1, iso2, iso3 et iso4 du complexe de formule (II) par UHPLC**

Un appareil UHPLC constitué d'un système de pompage, d'un injecteur, d'une colonne chromatographique, d'un détecteur UV et d'une station de données est utilisé. La colonne chromatographique utilisée est une colonne UHPLC 150 x 2,1 mm -1,6 μm (colonne CORTECS® UPLC T3 de Waters).
- Phase mobile :
Voie A : 100% acétonitrile et Voie B : solution aqueuse d'$H_2SO_4$ (96 %) à 0,0005 % v/v
- Préparation des solutions essais :
Solution du complexe de formule (II) à 2 mg/mL dans de l'eau purifiée
- Conditions d'analyse :

| | |
|---|---|
| Température colonne | 40°C |
| Température échantillon | Température ambiante (20-25°C) |
| Débit | 0,3 mL/min |
| Volume d'injection | 1 μl |
| Détection UV | 200 nm |
| Durée d'analyse | 20 min |

- Gradient :

| Temps | % Acn | % $H_2SO_4$ 0,0005 % |
|---|---|---|
| 0 | 1 | 99 |
| 3 | 5 | 95 |
| 12 | 10 | 90 |
| 15 | 25 | 75 |
| 16 | 1 | 99 |
| 20 | 1 | 99 |

4 pics principaux sont obtenus. Le pic 4 de la trace UHPLC, à savoir l'iso4, correspond à un temps de rétention de 6,3 minutes.

**Mesures de relaxivité**

Les temps de relaxation $T_1$ et $T_2$ ont été déterminés par des procédures standards sur un appareil Minispec® mq20 (Bruker) à 20 Mhz (0,47 T), à 60 Mhz (1,41 T) et 37°C. Le temps de relaxation longitudinal $T_1$ est mesuré en utilisant une séquence d'Inversion Récupération et le temps de relaxation transverse $T_2$ est mesuré par une technique

CPMG (Carr-Purcell-Meiboom-Gill).

Les vitesses de relaxation $R_1$ (= $1/T_1$) et $R_2$ (= $1/T_2$) ont été calculées pour différentes concentrations en métal total (variant de 0,5 x $10^{-3}$ à 5 x $10^{-3}$ mole/L) en solution aqueuse à 37°C. La corrélation entre $R_1$ ou $R_2$ en fonction de la concentration est linéaire, et la pente représente la relaxivité $r_1$ ($R_1$/C) ou $r_2$ ($R_2$/C) exprimées en (1 / seconde) x (1/ mMole/L) soit ($mM^{-1}.s^{-1}$).

**Mesure de l'inertie cinétique des groupes d'isomères du complexe de formule (II) en milieu acide**

La dissociation des complexes de gadolinium présents dans les 4 massifs d'isomères iso1 à iso4 (C = 8.10-6 M) est étudiée à 37°C, pH 1,2, dans une solution d'acide chlorhydrique dans des conditions de cinétique de pseudo premier ordre sans contrôle de la force ionique en suivant la libération du gadolinium dans la solution. La quantité de gadolinium libre a été déterminée par spectrométrie à 654 nm après ajout d'une solution d'Arsenazo III (C = 5,3.10-4 M).

Les temps de demi-vie ($T_{1/2}$) qui ont été déterminées pour chacun des groupes d'isomères sont rappelés dans le tableau ci-dessous :

| Groupes d'isomères | $T_{1/2}$ (pH 1,2 - 37°C) |
|---|---|
| Iso1 | 18 heures |
| Iso2 | 6 heures |
| Iso3 | 8 jours |
| Iso4 | 27 jours |

**Etude de dégradation en conditions basiques des groupes d'isomères du complexe de formule (II)**

Le complexe de formule (II) sera appelé PA dans la suite de cet exemple.

Les cinétiques de dégradation des massifs d'isomères iso1 à iso4, désignés par le terme générique isoX sont évaluées par mesure de la pureté HPLC et par le suivi de l'aire de chaque massif d'isomères au cours du temps. Les grandeurs mesurées sont ainsi : - $P_{HPLC}$ (temps), et

$$\frac{Aire_{IsoX}(t)}{Aire_{IsoX}(t_0)}$$

Les conditions de dégradation choisies sont les suivantes : [PA] = 1 mM dans la soude à 0,1 N. Dans ces conditions de dilution, l'impact de la dégradation du PA sur le milieu expérimental est faible. Les produits de dégradation ne modifient pas le pH du milieu, ce paramètre étant critique dans l'étude des cinétiques de dégradation. Ceci est confirmé expérimentalement par la mesure de pH initial et en fin de dégradation (72 heures, 37°C) :

| | Solution de PA |
|---|---|
| pH de NaOH 0,1 N ($T_0$) | 12.9 |
| pH (après 72 heures à 37°C) | 12,8 |

Le mode de préparation des solutions est décrit ci-après :

- peser environ 0,05 g de chaque produit qsp 10 mL de l'eau mQ, pour obtenir une solution A telle que $[PA]_A$ = 5 mM,
- dilution : 2 mL de solution A qsp 10 mL NaOH (0,1 N), pour obtenir une solution B telle que $[PA]_B$ = 1 mM et [NaOH] = 0.08 M,
- aliquote des solutions dans des flacons HPLC, et
- incubation des flacons HPLC contenant les solutions de PA dans NaOH à la température de l'étude (37°C).

Pour chaque point, un aliquote est prélevé et analysé en HPLC sans dilution de l'échantillon (méthode acétate d'ammonium).

Les résultats obtenus sont reportés en Figure 1.

## Préparation de l'hexaester de butyle de formule (VI)

Dans un réacteur, 184 kg (570 moles) de 2-bromoglutarate de dibutyle et 89 kg (644 moles) de carbonate de potassium sont mélangés et chauffés à 55-60°C. Une solution aqueuse de 29,4 kg (143 moles) de pyclène dans 24 kg d'eau est ajoutée à la préparation précédente. Le mélange réactionnel est maintenu à 55-60°C puis est chauffé à reflux une dizaine d'heures. Après réaction, le milieu est refroidi, dilué avec 155 kg de toluène puis lavé avec 300 litres d'eau. L'hexaester de butyle est extrait en phase aqueuse avec 175 kg (1340 moles) d'acide phosphorique (75%). Il est ensuite lavé 3 fois avec 150 kg de toluène. L'hexaester de butyle est reextrait en phase toluènique par dilution avec 145 kg de toluène et 165 kg d'eau suivie d'une basification à la soude 30 % (m/m) pour atteindre un pH de 5-5,5. La phase aqueuse inférieure est éliminée. L'hexaester de butyle est obtenu par concentration à sec sous vide à 60°C avec un rendement d'environ 85 %.

## Préparation de l'hexaacide de formule (III)

Dans un réacteur, 113 kg (121 moles) d'hexaester de butyle sont chargés ainsi que 8 kg d'éthanol. Le milieu est porté à 55+/-5°C puis 161 kg (1207.5 moles) de soude 30 % (m/m) sont coulés en 3 heures. Le mélange réactionnel est maintenu à cette température pendant une vingtaine d'heures. Le butanol est ensuite retiré par décantation du milieu réactionnel. L'hexaacide de formule (III) obtenu sous forme de sel de sodium est dilué avec de l'eau pour obtenir une solution aqueuse d'environ 10 % (m/m). Cette solution est traitée sur une résine cationique acide. L'hexaacide de formule (III) en solution aqueuse est obtenu avec un rendement d'environ 90 % et une pureté de 95 %.

## Préparation du complexe de gadolinium d'hexaacide de formule (I)

- ***Protocole Expérimental***

• Complexation et isomérisation

*- Sans acide acétique*

Dans un réacteur 418 kg (117 kg d'hexaacide de formule (III) pur / 196 moles) d'une solution aqueuse d'hexaacide de formule (III) à 28 % en poids sont chargés. Le pH de la solution est ajusté à 2,7 par ajout d'acide chlorhydrique, puis 37 kg (103,2 moles) d'oxyde de gadolinium sont ajoutés. Le milieu réactionnel est chauffé à 100-102°C pendant 48H pour atteindre la répartition isomérique attendue de l'hexaacide de formule (III).

*- Avec acide acétique*

De l'oxyde de gadolinium (0,525 éq. molaire) est mis en suspension dans une solution d'hexaacide de formule (III) à 28,1 % massique.

L'acide acétique 99-100 % (50 % massique / hexaacide de formule (III) pur) est coulé sur le milieu à température ambiante.

Le milieu est chauffé à reflux puis distillation jusqu'à 113°C en masse en rechargeant le milieu avec de l'acide acétique au fur et à mesure de l'élimination de l'eau. Arrivé à 113°C, on rajoute la quantité suffisante d'acide acétique afin d'arriver au volume de départ.

Le milieu est maintenu à 113°C pendant une nuit.

• Cristallisation, Recristallisation

*- Cristallisation*

Le complexe de gadolinium d'hexaacide de formule (I) en solution est refroidi à 40°C, l'amorce est ajoutée, on laisse en contact pendant au moins 2h. Il est ensuite isolé par filtration à 40°C et lavé avec de l'eau osmosée.

*- Recristallisation*

180 kg du complexe de gadolinium d'hexaacide de formule (I) obtenu précédemment (extrait sec à environ 72%) sont mis en suspension dans 390 kg d'eau. Le milieu est chauffé à 100°C pour solubiliser le produit, puis refroidi à 80°C pour être amorcé en ajoutant un peu d'amorce. Après refroidissement à température ambiante le complexe de gadolinium d'hexaacide de formule (I) est isolé par filtration et séchage.

• Décomplexation sélective

Le produit sec est chargé dans le réacteur avec de l'eau osmosée/ à 20°C. La masse d'eau ajoutée est égale au double de la masse de complexe de gadolinium d'hexaacide de formule (I) théorique. De la soude 30,5 % (m/m) (6,5 éq) est coulée sur le milieu à 20°C. On laisse le milieu en contact à 50°C à la fin de l'ajout de NaOH pendant 16h00. Le milieu est refroidi à 25°C et le produit filtré sur un lit de Clarcel.

- ***Teneur en excès diastéréoisomérique comprenant un mélange de diastéréoisomères I-RRR et I-SSS***

Le rapport dans lequel les différents isomères du complexe de formule (I) sont présents au sein du mélange de diastéréoisomères dépend des conditions dans lesquelles sont effectuées les étapes de complexation et d'isomérisation, comme cela apparaît dans le tableau 3 ci-dessous.

Tableau 3 : teneur en le mélange I-RRR et I-SSS en fonction des conditions de complexation / isomérisation

| *pH* | *Température* | *Teneur en hexaacide de formule (III)* | *Durée* | *Excès diastéréoisomérique comprenant un mélange I-RRR et I-SSS* |
|---|---|---|---|---|
| 5,7 | 80°C | 40% | 3h | 19% |
| 3,5 | 90°C | 50% | 10h | 49% |
| 3,0 | 101°C | 40% | 10h | 68% |
| 2,7 | 101°C | 28% | 48h | 98,04% |

Les étapes additionnelles de recristallisation et décomplexation sélective permettent d'augmenter l'excès diastéréoisomérique en le mélange I-RRR et I-SSS (voir tableau 4).

Tableau 4 : teneur en excès diastéréoisomérique comprenant un mélange I-RRR et I-SSS après cristallisation / recristallisation / décomplexation sélective

| | *Après la 1$^{ère}$ cristallisation* | *Après recristallisation* | *Après décomplexation sélective* |
|---|---|---|---|
| *Excès diastéréoisomérique comprenant un mélange I-RRR et I-SSS* | 98,04 % | 99,12 % | 99,75 % |

**Préparation du complexe de formule (II)**

Dans un réacteur, 90 kg (119 moles) du complexe d'hexaacide de formule (I) et 650 kg de méthanol sont chargés. Le mélange est refroidi à environ 0°C puis 111 kg (252 moles) d'une solution d'acide chlorhydrique méthanolique (8,25% d'HCI dans le méthanol) sont coulés en maintenant la température à 0°C. Le milieu réactionnel est porté à température ambiante puis est maintenu sous agitation pendant 16 heures. Après refroidissement à 0-5°C, 120 kg (1319 moles) d'3-amino-1,2-propanediol sont ajoutés. Le milieu réactionnel est ensuite chauffé en distillant le méthanol sous vide jusqu'à atteindre une température de 60-65°C. Le concentrât est maintenu pendant 16 heures à cette température sous vide. En fin de contact, le milieu est dilué avec 607 kg d'eau en refroidissant à température ambiante. La solution du complexe de formule (II) brut est neutralisée avec de l'acide chlorhydrique 20 % (m/m). 978,6 kg de solution sont ainsi obtenus, avec une concentration de 10,3 %, représentant 101 kg de matière. Le rendement obtenu est de 86.5 %.

**Essais de conversion d'isomères à partir des complexes de formule (II)**

Les isomères du complexe de formule (II) ont été synthétisés à partir des groupes d'isomères isoA, isoB, isoC et isoD du complexe d'hexaacide de formule (I) isolés par HPLC préparative. Les 4 groupes d'isomères ont été isolés puis amidifiés par du 3-amino-1,2-propanediol (APD) R et S. On obtient ainsi 8 isomères :

isoA + APD(R) et isoA + APD(S),

isoB + APD(R) et isoB + APD(S),

isoC + APD(R) et isoC + APD(S), et

isoD + APD(R) et isoD + APD(S).

Chacun de ces isomères a été placé dans les conditions permettant l'isomérisation du complexe de gadolinium d'hexaacide de formule (I).

Ainsi, on prépare une solution d'HCl à pH 3 en diluant 1 mL d'HCl 1 N dans 1 litre d'eau. Les isomères sont ajoutés à une concentration de 1 mM dans la solution d'HCl à pH 3. 10 mg de poudre sont dissous dans 10 mL de cette solution. Les 8 solutions obtenues sont chauffées à 100°C puis analysées à $T_0$ et à $T_0$ + 23 heures par HPLC.

Dans le tableau suivant, sont reportés les % de pureté mesurés par HPLC.

| | isoA + APD(S) | isoB + APD(S) | isoC + APD(S) | isoD + APD(S) |
|---|---|---|---|---|
| $T_0$ | 95,4 % | 92,3 % | 91 % | 98,6 % |
| $T_0$ + 23 h | 86% | 83% | 84 % | 92 % |

La perte de pureté est due à la dégradation chimique (hydrolyse des fonctions amide) du produit du fait des conditions imposées par la réaction d'isomérisation.

Comme les conditions permettant l'isomérisation des différents composés induisent une forte dégradation chimique des produits via l'hydrolyse des fonctions amide, l'isomérisation ne peut être réalisée de manière propre et sélective directement sur le complexe de formule (II) obtenu selon le procédé décrit dans le brevet EP 1 931 673.

**Revendications**

**1.** Procédé de préparation du complexe de formule (II) suivante :

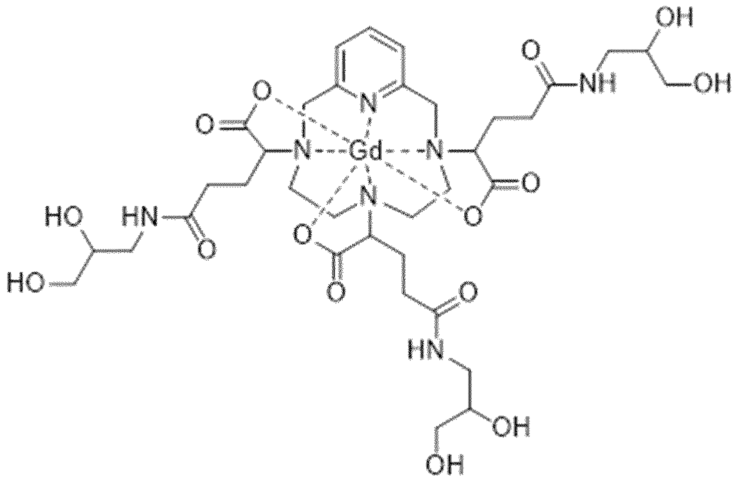

(II)

constitué d'au moins 80 % d'un excès diastéréoisomérique comprenant un mélange d'isomères II-RRR et II-SSS de formules :

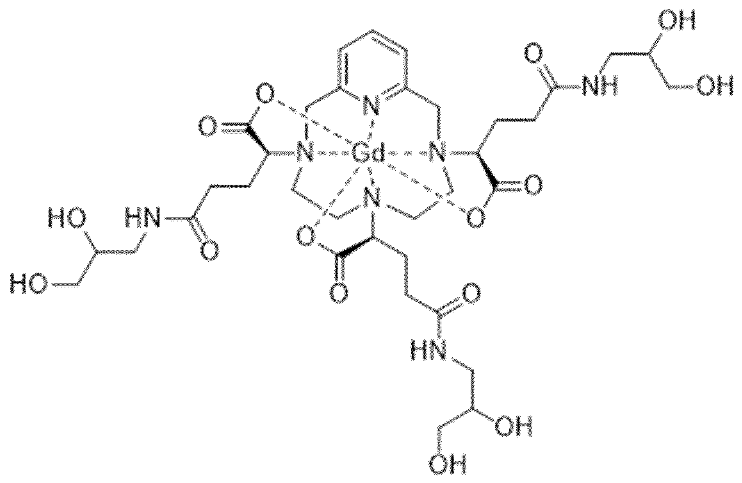

(II-SSS),

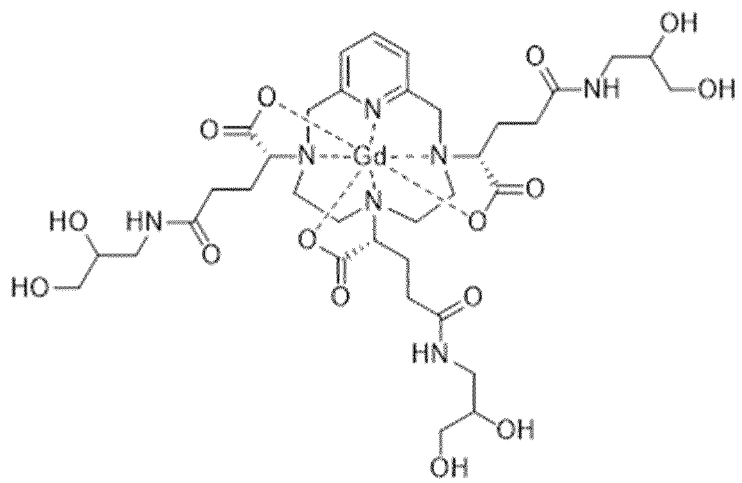

(II-RRR),

comprenant les étapes successives suivantes :

a) Complexation de l'hexaacide de formule (III) suivante :

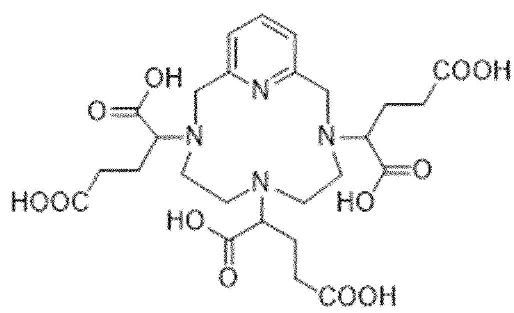

(III)

avec du gadolinium pour obtenir le complexe de gadolinium d'hexaacide de formule (I) suivante :

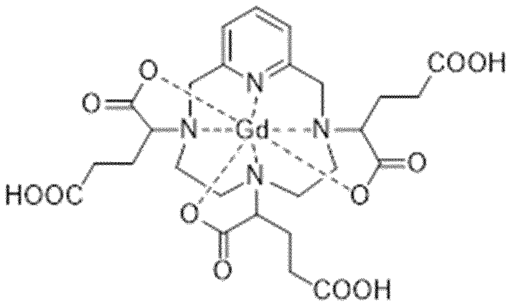

(I),

b) Isomérisation par chauffage du complexe de gadolinium d'hexaacide de formule (I) dans une solution aqueuse à pH compris entre 2 et 4, pour obtenir un complexe diastéréoisomériquement enrichi constitué d'au moins 80% d'un excès diastéréoisomérique comprenant un mélange des isomères I-RRR et I-SSS dudit complexe de gadolinium d'hexaacide de formule (I), de formules :

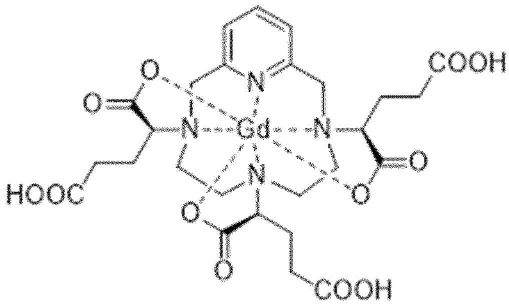

(I-SSS),

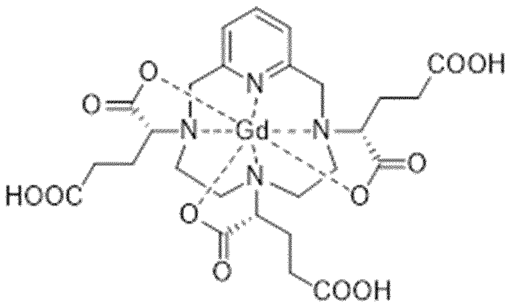

(I-RRR),

et

c) Formation, à partir du complexe diastéréoisomériquement enrichi obtenu à l'étape b), du complexe de formule (II), par réaction avec le 3-amino-1,2-propanediol.

2. Procédé selon la revendication 1, **caractérisé en ce que** le complexe de gadolinium d'hexaacide de formule (I) formé au cours de l'étape a) est directement soumis à l'étape b) d'isomérisation sans être isolé ou purifié.

3. Procédé selon la revendication 1 ou 2, **caractérisé en ce que** la solution aqueuse de l'étape b) comprend de l'acide acétique.

4. Procédé selon l'une quelconque des revendications 1 à 3, **caractérisé en ce que** l'étape b) est réalisée à une température comprise entre 100°C et 120°C, typiquement pendant une durée comprise entre 12h et 48h.

5. Procédé selon l'une quelconque des revendications 1 à 4, **caractérisé en ce que** l'étape b) comprend les étapes successives suivantes :

b1) Isomérisation par chauffage du complexe de gadolinium d'hexaacide de formule (I) dans une solution

aqueuse à pH compris entre 2 et 4, pour obtenir un complexe diastéréoisomériquement enrichi constitué d'au moins 80% de l'excès diastéréoisomérique comprenant le mélange des isomères I-RRR et I-SSS dudit complexe de gadolinium d'hexaacide de formule (I), et

b2) Isolement par cristallisation dudit complexe diastéréoisomériquement enrichi, de préférence par cristallisation par ensemencement.

6. Procédé selon la revendication 5, **caractérisé en ce que** le complexe diastéréoisomériquement enrichi de l'étape b) isolé par cristallisation est purifié par recristallisation, pour obtenir un complexe diastéréoisomériquement enrichi et purifié.

7. Procédé selon la revendication 5 ou 6, **caractérisé en ce que** le complexe diastéréoisomériquement enrichi de l'étape b) est encore enrichi par décomplexation sélective des diastéréoisomères du complexe de formule (I) autres que les diastéréoisomères I-RRR et I-SSS, *i.e.* par décomplexation sélective des diastéréoisomères I-RSS, I-SRR, I-RSR, I-SRS, I-RRS et I-SSR.

8. Procédé selon l'une quelconque des revendications 1 à 7, **caractérisé en ce que** le complexe diastéréoisomériquement enrichi obtenu à l'issue de l'étape b) selon l'une quelconque des variantes décrites ci-dessus présente au moins 85 % de l'excès diastéréoisomérique comprenant le mélange des isomères I-RRR et I-SSS.

9. Procédé selon l'une quelconque des revendications 1 à 8, **caractérisé en ce que** le complexe diastéréoisomériquement enrichi obtenu à l'issue de l'étape b) selon l'une quelconque des variantes décrites ci-dessus présente au moins 90 % de l'excès diastéréoisomérique comprenant le mélange des isomères I-RRR et I-SSS.

10. Procédé selon l'une quelconque des revendications 1 à 9, **caractérisé en ce que** l'étape c) comprend les étapes successives suivantes :

c1) formation d'un triester de formule (VIII),

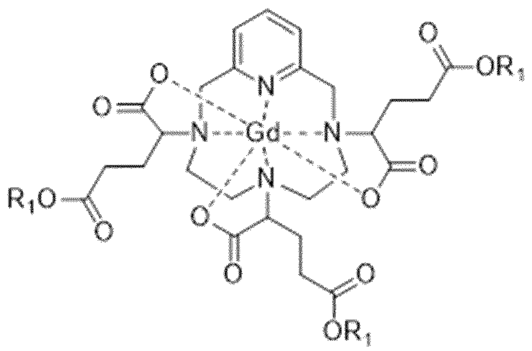

(VIII)

dans laquelle $R_1$ représente un groupe ($C_1$-$C_6$)alkyle, notamment par réaction dans l'alcool de formule $R_1OH$ en présence d'un acide tel que l'acide chlorhydrique, et

c2) aminolyse du triester de formule (VIII) avec le 3-amino-1,2-propanediol, notamment dans l'alcool de formule $R_1OH$ en présence d'un acide tel que l'acide chlorhydrique.

11. Procédé selon la revendication 10, **caractérisé en ce que** le triester de formule (VIII) n'est pas isolé entre les étapes c1) et c2).

12. Procédé selon la revendication 10 ou 11, **caractérisé en ce que** $R_1$ représente un groupe méthyle.

13. Procédé selon l'une quelconque des revendications 1 à 12, **caractérisé en ce que** l'étape c) comprend les étapes successives suivantes :

c1) formation d'un triester de méthyle de formule (IV),

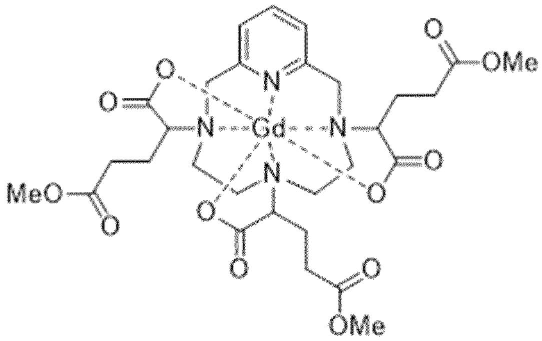

(IV)

notamment par réaction dans du méthanol en présence d'un acide tel que l'acide chlorhydrique, et

c2) aminolyse du triester de méthyle de formule (IV) avec le 3-amino-1,2-propanediol, dans du méthanol en présence d'un acide tel que l'acide chlorhydrique, au cours de laquelle le méthanol est éliminé par distillation sous vide, jusqu'à atteindre une température supérieure à 55°C, le milieu réactionnel étant maintenu à cette température sous vide pendant une durée typiquement supérieure à 5h avant d'être refroidi à température ambiante et dilué avec de l'eau.

**14.** Procédé selon l'une quelconque des revendications 1 à 13, **caractérisé en ce que** l'hexaacide de formule (III) telle que définie dans la revendication 1 est obtenu par alkylation du pyclène de formule (V) :

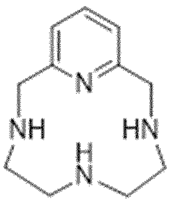

(V)

avec le 2-bromoglutarate de dibutyle, pour obtenir l'hexaester de butyle de formule (VI)

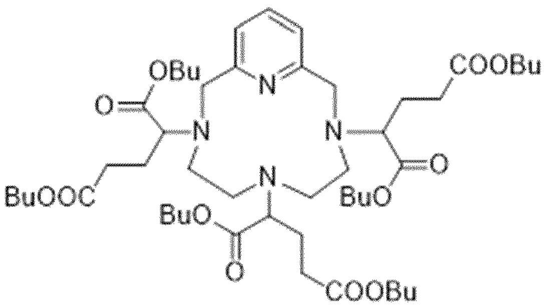

(VI),

suivie d'une étape d'hydrolyse, conduisant audit hexaacide de formule (III).

## Patentansprüche

**1.** Verfahren zur Herstellung des Komplexes der folgenden Formel (II):

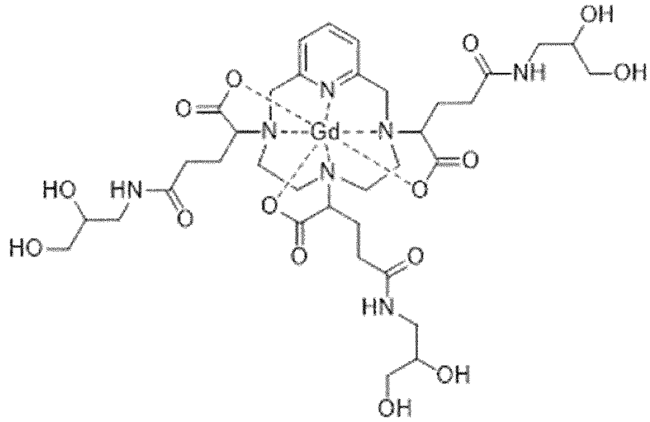

(II)

bestehend aus mindestens 80 % eines Diastereoisomerenüberschusses, umfassend ein Gemisch von Isomeren II-RRR und II-SSS der Formeln:

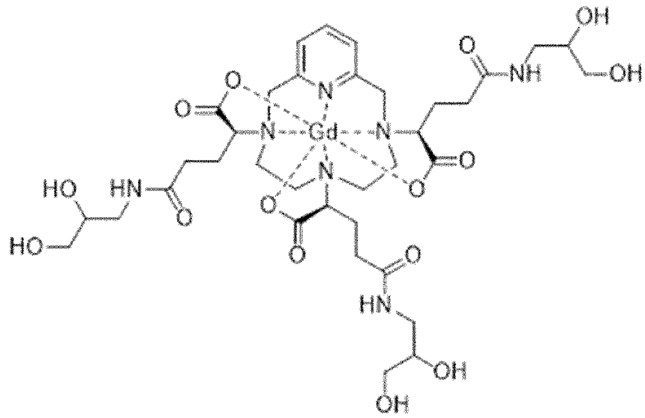

(II-SSS),

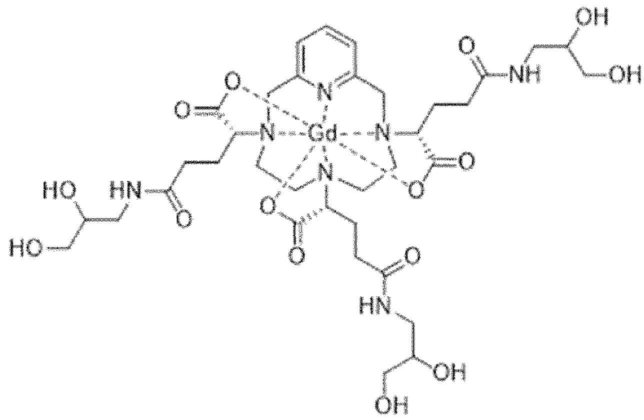

(II-RRR),

das die folgenden aufeinanderfolgenden Schritte umfasst:

a) Komplexierung der Hexasäure der folgenden Formel (III):

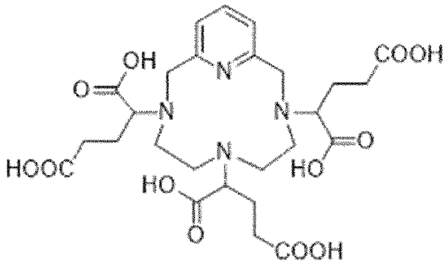

(III)

mit Gadolinium unter Erhalt des Hexasäure-Gadolinium-Komplexes der folgenden Formel (I):

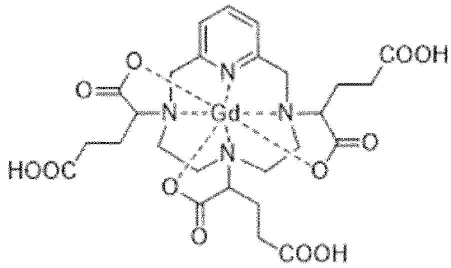

(I),

b) Isomerisierung des Hexasäure-Gadolinium-Komplexes der Formel (I) in einer wässrigen Lösung bei einem pH-Wert zwischen 2 und 4 durch Erhitzen unter Erhalt eines diastereomerenangereicherten Komplexes, bestehend aus mindestens 80 % eines Diastereoisomerenüberschusses, umfassend ein Gemisch von Isomeren I-RRR und I-SSS des Hexasäure-Gadolinium-Komplexes der Formel (I) der Formeln:

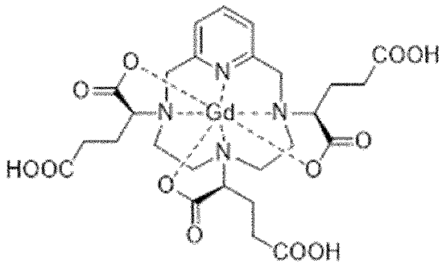

(I-SSS),

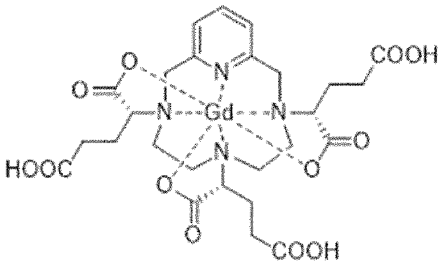

(I-RRR),

und

c) Bildung des Komplexes der Formel (II) ausgehend von dem in Schritt b) erhaltenen diastereomerenangereicherten Komplex durch Umsetzung mit 3-Amino-1,2-propandiol.

2. Verfahren nach Anspruch 1, **dadurch gekennzeichnet, dass** der in Schritt a) erhaltene Hexasäure-Gadolinium-Komplex der Formel (I) ohne Isolierung oder Reinigung direkt dem Isomerisierungsschritt b) unterworfen wird.

3. Verfahren nach Anspruch 1 oder 2, **dadurch gekennzeichnet, dass** die wässrige Lösung von Schritt b) Essigsäure umfasst.

4. Verfahren nach einem der Ansprüche 1 bis 3, **dadurch gekennzeichnet, dass** der Schritt b) bei einer Temperatur

zwischen 100 und 120 °C durchgeführt wird, typischerweise über eine Dauer zwischen 12 h und 48 h.

5. Verfahren nach einem der Ansprüche 1 bis 4, **dadurch gekennzeichnet, dass** der Schritt b) die folgenden aufeinanderfolgenden Schritte umfasst:

b1) Isomerisierung des Hexasäure-Gadolinium-Komplexes der Formel (I) in einer wässrigen Lösung bei einem pH-Wert zwischen 2 und 4 durch Erhitzen unter Erhalt eines diastereomerenangereicherten Komplexes, bestehend aus mindestens 80 % eines Diastereoisomerenüberschusses, umfassend ein Gemisch von Isomeren I-RRR und I-SSS des Hexasäure-Gadolinium-Komplexes der Formel (I) und
b2) Isolierung des diastereomerenangereicherten Komplexes durch Kristallisation, vorzugsweise durch Impfkistallisation.

6. Verfahren nach Anspruch 5, **dadurch gekennzeichnet, dass** der durch Kristallisation isolierte diastereomerenangereicherte Komplex aus Schritt b) durch Umkristallisation unter Erhalt eines diastereomerenangereicherten und gereinigten Komplexes gereinigt wird.

7. Verfahren nach Anspruch 5 oder 6, **dadurch gekennzeichnet, dass** der diastereomerenangereicherte Komplex aus Schritt b) durch selektive Dekomplexierung der Diastereomere des Komplexes der Formel (I), die von den Diastereoisomeren I-RRR und I-SSS verschieden sind, d. h. durch selektive Dekomplexierung der Diastereomere I-RSS, I-SRR, I-RSR, I-SRS, I-RRS und I-SSR, weiter angereichert wird.

8. Verfahren nach einem der Ansprüche 1 bis 7, **dadurch gekennzeichnet, dass** der am Ende von Schritt b) gemäß einer der oben beschriebenen Varianten erhaltene diastereomerenangereicherte Komplex einen Diastereomerenüberschuss, umfassend das Gemisch der Isomere I-RRR und I-SSS, von mindestens 85 % aufweist.

9. Verfahren nach einem der Ansprüche 1 bis 8, **dadurch gekennzeichnet, dass** der am Ende von Schritt b) gemäß einer der oben beschriebenen Varianten erhaltene diastereomerenangereicherte Komplex einen Diastereomerenüberschuss, umfassend das Gemisch der Isomere I-RRR und I-SSS, von mindestens 90 % aufweist.

10. Verfahren nach einem der Ansprüche 1 bis 9, **dadurch gekennzeichnet, dass** der Schritt c) die folgenden aufeinanderfolgenden Schritte umfasst:

c1) Bildung eines Triesters der Formel (VIII),

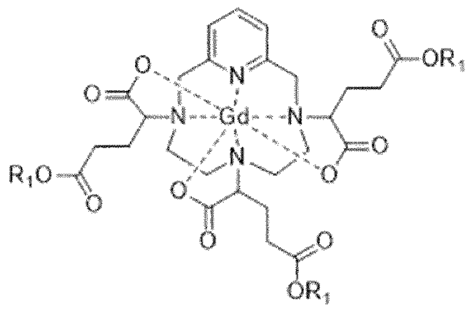

(VIII)

wobei $R_1$ für eine ($C_1$-$C_6$) -Alkylgruppe steht, insbesondere durch Umsetzung in dem Alkohol der Formel $R_1OH$ in Gegenwart einer Säure wie Salzsäure, und
c2) Aminolyse des Triesters der Formel (VIII) mit 3-Amino-1,2-propandiol, insbesondere in dem Alkohol der Formel $R_1OH$ in Gegenwart einer Säure wie Salzsäure.

11. Verfahren nach Anspruch 10, **dadurch gekennzeichnet, dass** der Triester der Formel (VIII) zwischen den Schritten c1) und c2) nicht isoliert wird.

12. Verfahren nach Anspruch 10 oder 11, **dadurch gekennzeichnet, dass** $R_1$ für eine Methylgruppe steht.

13. Verfahren nach einem der Ansprüche 1 bis 12, **dadurch gekennzeichnet, dass** der Schritt c) die folgenden aufeinanderfolgenden Schritte umfasst:

c1) Bildung eines Methyltriesters der Formel (IV),

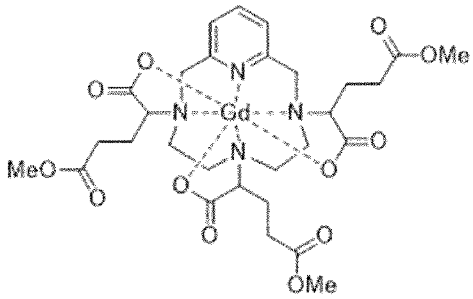

(IV)

insbesondere durch Umsetzung in Methanol in Gegenwart einer Säure wie Salzsäure, und
c2) Aminolyse des Methyltriesters der Formel (IV) mit 3-Amino-1,2-propandiol in Methanol in Gegenwart einer Säure wie Salzsäure, in deren Verlauf das Methanol durch Destillation unter Vakuum bis zum Erreichen einer Temperatur von mehr als 55 °C entfernt wird, wobei das Reaktionsmedium über einen Zeitraum von typischerweise mehr als 5 h bei dieser Temperatur unter Vakuum gehalten und dann auf Umgebungstemperatur abgekühlt und mit Wasser verdünnt wird.

**14.** Verfahren nach einem der Ansprüche 1 bis 13, **dadurch gekennzeichnet, dass** die Hexasäure der Formel (III) gemäß Anspruch 1 durch Alkylierung von Pyclen der Formel (V):

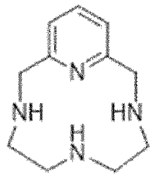
(V)

mit 2-Bromglutarsäuredibutylester unter Erhalt des Butylhexaesters der Formel (VI)

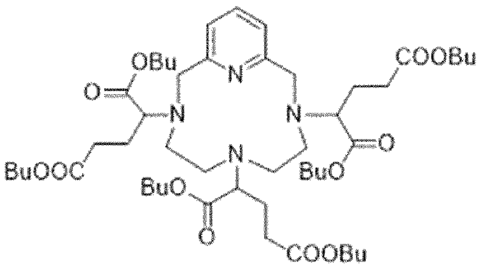
(VI)

und einen anschließenden Hydrolyseschritt, der zu der Hexasäure der Formel (III) führt, erhalten wird.

## Claims

**1.** Process for preparing the complex of formula (II) below:

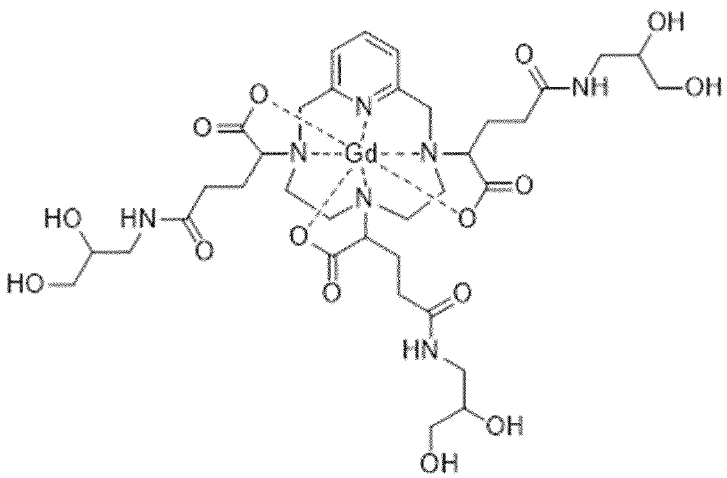

(II)

consisting of at least 80% of a diastereoisomeric excess comprising a mixture of the II-RRR and II-SSS isomers of formulae:

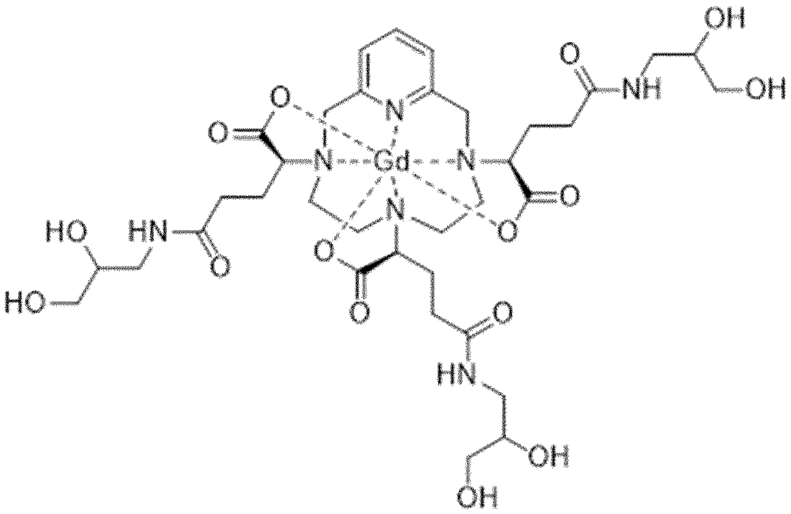

(II-SSS)

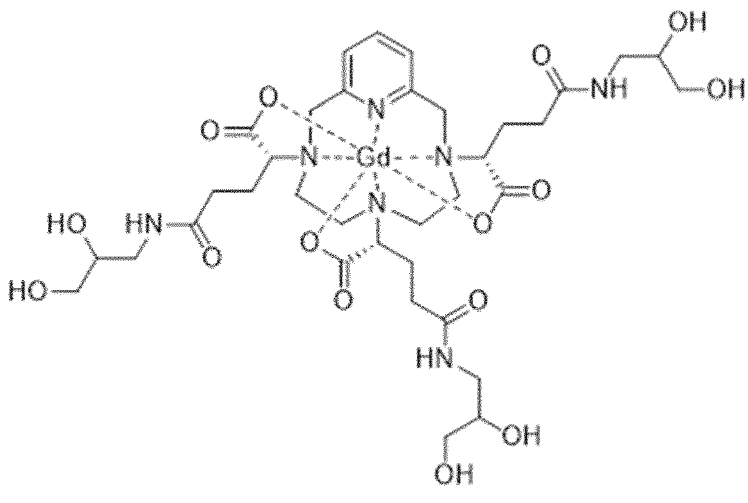

(II-RRR),

comprising the following successive steps:

a) Complexation of the hexaacid of formula (III) below:

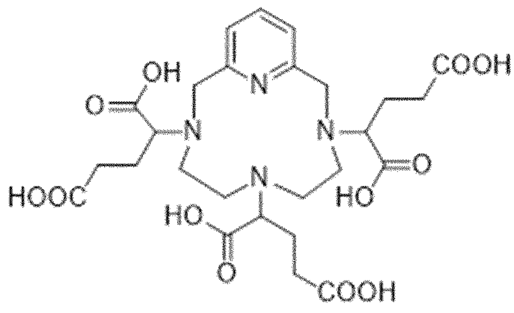

(III)

with gadolinium to obtain the hexaacid gadolinium complex of formula (I) below:

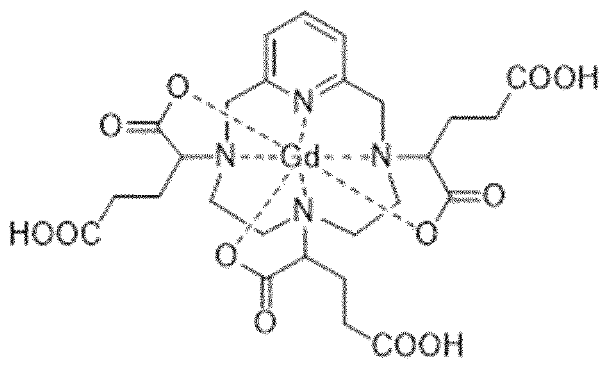

(I),

b) Isomerization by heating of the hexaacid gadolinium complex of formula (I) in an aqueous solution with a pH of between 2 and 4, to obtain a diastereoisomerically enriched complex consisting of at least 80% of a diastereoisomeric excess comprising a mixture of the I-RRR and I-SSS isomers of said hexaacid gadolinium complex of formula (I), of formulae:

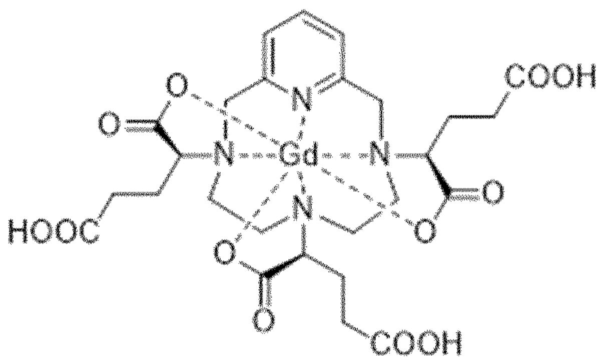

(I-SSS),

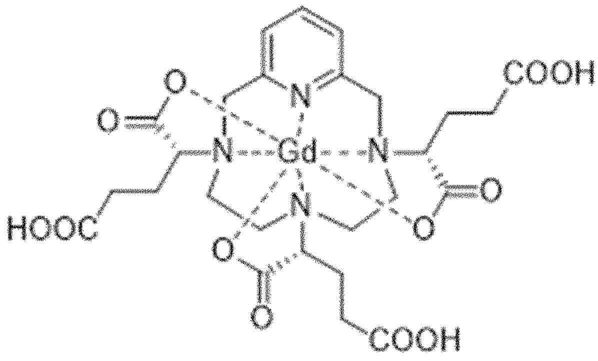

(I-RRR),

and

c) Formation, from the diastereoisomerically enriched complex obtained in step b), of the complex of formula (II), by reaction with 3-amino-1,2-propanediol.

2. Process according to Claim 1, **characterized in that** the hexaacid gadolinium complex of formula (I) formed in the course of step a) is directly subjected to the isomerization step b) without being isolated or purified.

3. Process according to Claim 1 or 2, **characterized in that** the aqueous solution of step b) comprises acetic acid.

4. Process according to any one of Claims 1 to 3, **characterized in that** step b) is performed at a temperature of between 100°C and 120°C, typically for a time of between 12 hours and 48 hours.

5. Process according to any one of Claims 1 to 4, **characterized in that** step b) comprises the following successive steps:

b1) Isomerization by heating the hexaacid gadolinium complex of formula (I) in an aqueous solution with a pH of between 2 and 4, to obtain a diastereoisomerically enriched complex consisting of at least 80% of the diastereoisomeric excess comprising the mixture of the I-RRR and I-SSS isomers of said hexaacid gadolinium complex of formula (I), and
b2) Isolation by crystallization of said diastereoisomerically enriched complex, preferably by crystallization by seeding.

6. Process according to Claim 5, **characterized in that** the diastereoisomerically enriched complex from step b) isolated by crystallization is purified by recrystallization, to obtain a diastereoisomerically enriched and purified complex.

7. Process according to Claim 5 or 6, **characterized in that** the diastereoisomerically enriched complex from step b) is further enriched by selective decomplexation of the diastereoisomers of the complex of formula (I) other than the I-RRR and I-SSS diastereoisomers, i.e. by selective decomplexation of the I-RSS, I-SRR, I-RSR, I-SRS, I-RRS and I-SSR diastereoisomers.

8. Process according to any one of Claims 1 to 7, **characterized in that** the diastereoisomerically enriched complex obtained on conclusion of step b) according to any one of the variants described above has at least 85% of the diastereoisomeric excess comprising the mixture of the I-RRR and I-SSS isomers.

9. Process according to any one of Claims 1 to 8, **characterized in that** the diastereoisomerically enriched complex obtained on conclusion of step b) according to any one of the variants described above has at least 90% of the diastereoisomeric excess comprising the mixture of the I-RRR and I-SSS isomers.

10. Process according to any one of Claims 1 to 9, **characterized in that** step c) comprises the following successive steps:

c1) formation of a triester of formula (VIII)

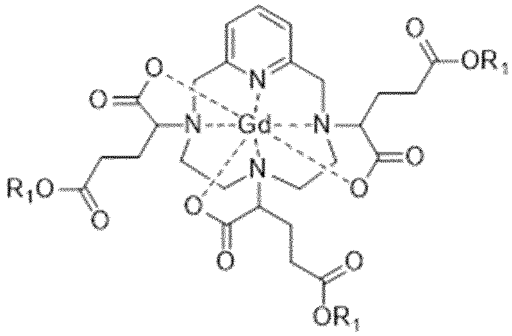

(VIII)

in which $R_1$ represents a ($C_1$-$C_6$) alkyl group, notably by reaction in the alcohol of formula $R_1OH$ in the presence of an acid such as hydrochloric acid, and
c2) aminolysis of the triester of formula (VIII) with 3-amino-1,2-propanediol, notably in the alcohol of formula $R_1OH$ in the presence of an acid such as hydrochloric acid.

**11.** Process according to Claim 10, **characterized in that** the triester of formula (VIII) is not isolated between steps c1) and c2).

**12.** Process according to Claim 10 or 11, **characterized in that** $R_1$ represents a methyl group.

**13.** Process according to any one of Claims 1 to 12, **characterized in that** step c) comprises the following successive steps:

c1) formation of a methyl triester of formula (IV)

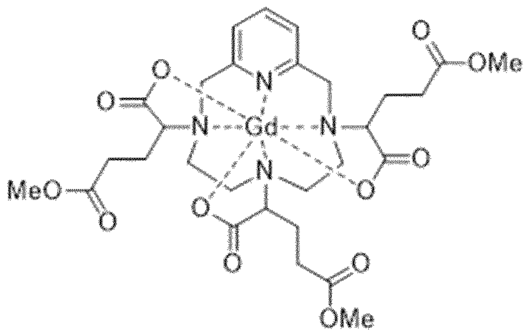

(IV)

notably by reaction in methanol in the presence of an acid such as hydrochloric acid, and
c2) aminolysis of the methyl triester of formula (IV) with 3-amino-1,2-propanediol in methanol in the presence of an acid such as hydrochloric acid, during which the methanol is removed by vacuum distillation, until a temperature of greater than 55°C is reached, the reaction medium being maintained at this temperature under vacuum for a time typically greater than 5 hours before being cooled to room temperature and diluted with water.

**14.** Process according to any one of Claims 1 to 13, **characterized in that** the hexaacid of formula (III) as defined in Claim 1 is obtained by alkylation of pyclene of formula (V):

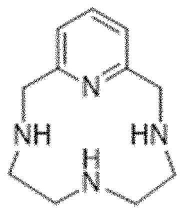
(V)

with dibutyl 2-bromoglutarate, to obtain the butyl hexaester of formula (VI)

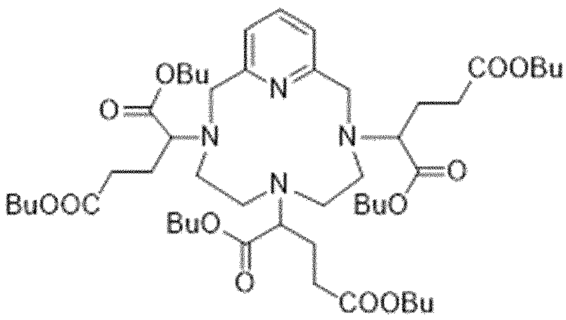
(VI),

followed by a step of hydrolysis, leading to said hexaacid of formula (III).

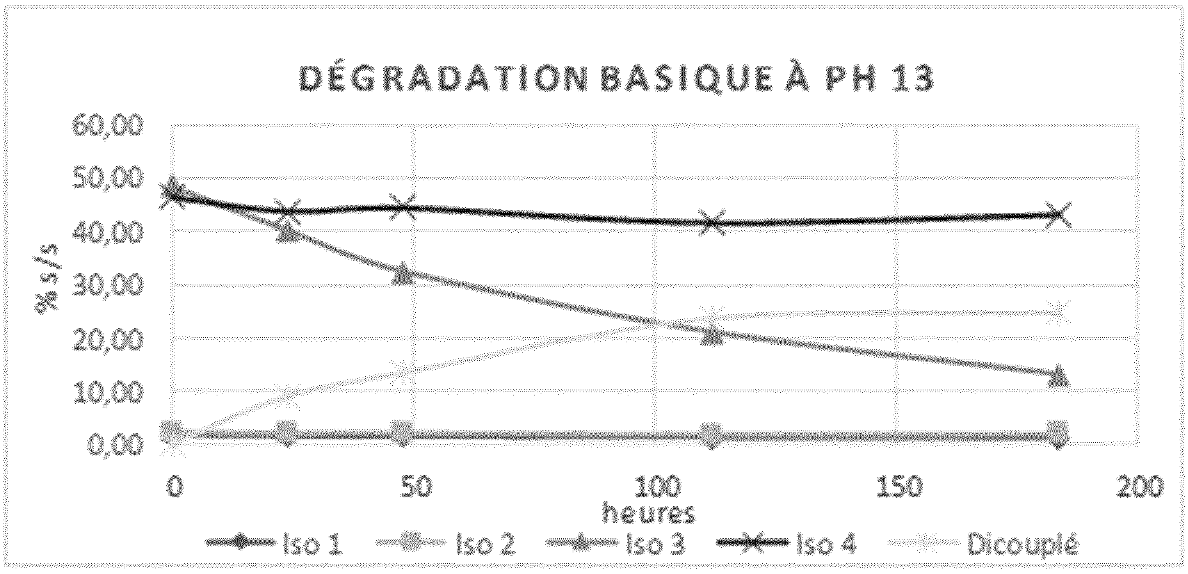
DÉGRADATION BASIQUE À PH 13
60,00
50,00
40,00
30,00
20,00
10,00
0,00
% s/s
0
50
100
150
200
heures
Iso 1
Iso 2
Iso 3
Iso 4
Dicouplé

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4647447 A
- EP 0438206 A
- EP 1931673 A
- EP 1931673 W
- WO 2014174120 A


### Littérature non-brevet citée dans la description


- **PIERRARD et al.** *Contrast Media Mol. Imaging,* 2008, vol. 3, 243-252
- **MOREAU et al.** *Dalton Trans.,* 2007, 1611-1620
- **HOUBEN-WEYL et al.** Synthesis of peptides and peptidomimetics. Thieme-Stuttgart, 2004, vol. E22a, 425-588
- **K. C. NADIMPALLY et al.** *Tetrahedron Letters,* 2011, vol. 52, 2579-2582